# EUROPEAN PATENT APPLICATION

(11) **EP 4 496 458 A2**
(43) Date of publication of application: **22.01.2025**
(21) Application number: 24208778.1
(22) Date of filing: 27.07.2020
(51) Int. Cl.: H10K 85/30

(54) **METAL COMPLEXES OF 3-(2,3,5-TRIFLUORO-6-(TRIFLUOROMETHYL)PYRIDIN-4-YL)PENTANE-2,4-DIONE AND SIMILAR LIGANDS AS SEMICONDUCTOR MATERIALS FOR USE IN ELECTRONIC DEVICES**

(62) Divisional of application: 20187943.4
(71) Applicant: Novaled GmbH, 01099 Dresden (DE)
(72) Inventor: UVAROV, Vladimir, 01099 Dresden (DE); NÜLLEN, Max Peter, 01099 Dresden (DE); HEGGEMANN, Ulrich, 01099 Dresden (DE); HUMMERT, Markus, 01099 Dresden (DE)
(74) Representative: Michalski Hüttermann & Partner Patentanwälte mbB

(57) **Abstract**

The present invention relates to compound represented by Formula (I), wherein M is a metal; L is a charge-neutral ligand, which coordinates to the metal M; n is an integer selected from 1 to 4, which corresponds to the oxidation number of M; m is an integer selected from 0 to 2; R¹, R² and R³ are substituents; a semiconductor material comprising at least one compound of formula (I), an semiconductor layer comprising at least one compound of formula (I) and an electronic device comprising at least one compound of formula (I).

## Description

### Technical Field

The present invention relates to a compound of formula (I), a semiconductor material comprising at least one compound of formula (I), an semiconductor layer comprising at least one compound of formula (I) and an electronic device comprising at least one compound of formula (I).

### Background Art

Electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode layer, a hole injection layer HIL, a hole transport layer HTL, an emission layer EML, an electron transport layer ETL, and a cathode layer, which are sequentially stacked on a substrate. In this regard, the HIL, the HTL, the EML, and the ETL are thin films formed from organic compounds.

When a voltage is applied to the anode and the cathode, holes injected from the anode move to the EML, via the HIL and HTL, and electrons injected from the cathode move to the EML, via the ETL. The holes and electrons recombine in the EML, to generate excitons. When the excitons drop from an excited state to a ground state, light is emitted. The injection and flow of holes and electrons should be balanced, so that an OLED having the above-described structure has low operating voltage, excellent efficiency and/or a long lifetime.

Performance of an organic light emitting diode may be affected by characteristics of the hole injection layer, and among them, may be affected by characteristics of the hole transport compound and the metal complexes which are contained in the hole injection layer.

US 2015200374 A relates to a hole injection layer consisting of quadratic planar mononuclear transition metal complexes such as copper 2+ complexes, for example, which are embedded into a hole-conducting matrix.

WO16188604 A1 relates to a composition at least one hole-transport or/and one hole-injection material and at least one metal complex as a p-dopant.

Performance of an organic light emitting diode may be affected by characteristics of the semiconductor layer, and among them, may be affected by characteristics of metal complexes which are also contained in the semiconductor layer.

There remains a need to improve performance of semiconductor materials, semiconductor layers, as well as electronic devices thereof, in particular to achieve improved operating voltage stability over time through improving the characteristics of the compounds comprised therein.

Further there remains a need to improve performance of electronic devices by providing hole injection layers with improved performance, in particular to achieve improved operating voltage through improving the characteristics of the hole injection layer and the electronic device.

Furthermore, there remains a need to provide hole injection layers which enable injection into adjacent layers comprising compounds with a HOMO level further away from vacuum level.

It is also objective to provide a hole injection layer comprising compounds which may be deposited through vacuum thermal evaporation under conditions suitable for mass production.

### DISCLOSURE

An aspect of the present invention provides a compound represented by Formula I: 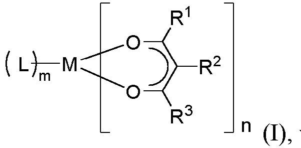 wherein
M is a metal;
L is a charge-neutral ligand, which coordinates to the metal M;
n is an integer selected from 1 to 4, which corresponds to the oxidation number of M;
m is an integer selected from 0 to 2;
R¹, R² and R³ are independently selected from H, D, substituted or unsubstituted C₁ to C₁₂ alkyl, substituted or unsubstituted C₁ to C₁₂ alkoxy, substituted or unsubstituted C₆ to C₂₄ aryl, and substituted or unsubstituted C₂ to C₂₄ heteroaryl group, wherein
   the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein
   the substituents are selected from halogen, F, Cl, CN, C₁ to C₆ alkyl, CF₃, OCH₃ and OCF₃;
wherein
   at least one R¹, R² and/or R³ is selected from a substituted C₂ to C₂₄ heteroaryl group, wherein at least one substituent is selected from halogen, F, Cl, CN, partially or fully fluorinated C₁ to C₆ alkyl, partially or fully fluorinated C₁ to C₆ alkoxy.

### Definitions

It should be noted that throughout the application and the claims any R¹, R², R³, L and M. always refer to the same moieties, unless otherwise noted.

In the present specification, when a definition is not otherwise provided, "substituted" refers to a substituted selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein the substituents are selected from halogen, F, Cl, CN, C₁ to C₆ alkyl, CF₃, OCH₃ and OCF₃.

In the present specification, "aryl group" and "aromatic rings" refers to a hydrocarbyl group which may be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under "heteroaryl" and "heteroaromatic", it is especially where suitable understood a group derived by formal abstraction of one ring hydrogen from a heterocyclic aromatic ring in a compound comprising at least one such ring.

The term "non-heterocycle" is understood to mean a ring or ring-system comprising no hetero-atom as a ring member.

The term "heterocycle" is understood to mean that the heterocycle comprises at least one ring comprising one or more hetero-atoms. A heterocycle comprising more than one ring means that all rings comprising a hetero-atom or at least one ring comprising a hetero atom and at least one ring comprising C-atoms only and no hetero atom.

Under heterocycloalkyl, it is especially where suitable understood a group derived by formal abstraction of one ring hydrogen from a saturated cycloalkyl ring in a compound comprising at least one such ring.

The term "aryl" having at least 9 C-atoms may comprise at least one fused aryl ring. The term "heteroaryl" having at least 9 atoms may comprise at least one fused heteroaryl ring fused with a heteroaryl ring or fused with an aryl ring.

The term "fused aryl rings" or "condensed aryl rings" is understood the way that two aryl rings are considered fused or condensed when they share at least two common sp²-hybridized carbon atoms.

The term "fused ring system" is understood to mean a ring system wherein two or more rings share at least two atoms.

The term "5-, 6- or 7-member ring" is understood to mean a ring comprising 5, 6 or 7 atoms. The atoms may be selected from C and one or more hetero-atoms.

In the present specification, the single bond refers to a direct bond.

In the present specification, when a definition is not otherwise provided, "substituted" refers to one substituted with a H, deuterium, C₁ to C₁₂ alkyl, unsubstituted C₆ to C₁₈ aryl, and unsubstituted C₂ to C₁₈ heteroaryl.

In the present specification "substituted aryl" refers for example to a C₆ to C₂₄ aryl or C₆ to C₁₈ aryl that is substituted with one or more substituents, wherein the substituent may be substituted with none, one or more substituents.

Correspondingly, in the present specification "substituted heteroaryl substituted" refers to a substitution with one or more substituents, which themselves may be substituted with one or more substituents.

In the present specification, when a definition is not otherwise provided, a substituted heteroaryl group with at least 2 C-ring atoms may be substituted with one or more substituents. For example, a substituted C₂ heteroaryl group may have 1 or 2 substituents.

A substituted aryl group with at least 6 ring atoms may be substituted with 1, 2, 3, 4 or 5 substituents.

A substituted heteroaryl group may comprise at least 6 ring atoms. A substituted heteroaryl group that may comprise at least 6 ring atoms may be substituted with 1, 2, 3 or 4 substituents, if the heteroaryl group comprises one hetero atom and five C-atoms, or it may be substituted with 1, 2 or 3 substituents, if the heteroaryl group with at least 6 ring atoms comprises two hetero atom and four C-atoms, or may be substituted with 1 or 2 substituents, if the heteroaryl group with at least 6 ring atoms comprises three hetero atoms and three C-atoms, wherein the substituent is bonded to the C-ring atoms only.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, tert-butyl, cyclohexyl.

Specific examples of the alkyl group may be a methyl group, an ethyl group, a propyl group, an iso-propyl group, a butyl group, an iso-butyl group, a sec-butyl group, a tert-butyl group, a pentyl group, a branched pentyl group, a hexyl group, a cyclopropyl group, a cyclobutyl group, a cyclopentyl group, a cyclohexyl group, an adamantly group and the like.

In the present specification, when a definition is not otherwise provided, a "substituted alkyl group" may refer to a linear, branched or cyclic substituted saturated aliphatic hydrocarbyl group. The substituted alkyl group may be a linear, branched or cyclic C₁ to C₁₂ alkyl group. More specifically, the substituted alkyl group may be a linear, branched or cyclic substituted C₁ to C₁₀ alkyl group or a linear, branched or cyclic substituted C₁ to C₆ alkyl group. For example, a linear, branched or cyclic substituted C₁ to C₄ alkyl group includes 1 to 4 carbons in the alkyl chain, and may be selected from methyl, ethyl, propyl, iso-propyl, n-butyl, iso-butyl, sec-butyl, tert-butyl and cyclohexyl. The substituents may be selected from halogen, F, Cl, CN, OCH₃, OCF₃.

The term "hetero" is understood the way that at least one carbon atom, in a structure which may be formed by covalently bound carbon atoms, is replaced by another polyvalent atom. Preferably, the heteroatoms are selected from B, Si, N, P, O, S; further preferred from N, P, O, S and most preferred N.

In the present specification, when a substituent is not named, the substituent may be a H.

The term "charge-neutral" means that the group L is overall electrically neutral.

In the context of the present invention, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms anode, anode layer and anode electrode are used synonymously.

The term "at least two anode sub-layers" is understood to mean two or more anode sub-layers, for example two or three anode sub-layers.

The terms cathode, cathode layer and cathode electrode are used synonymously.

The term "hole injection layer" is understood to mean a layer which improves charge injection from the anode layer into further layers in the electronic device or from further layers of the electronic device into the anode.

The term "hole transport layer" is understood to mean a layer which transports holes between the hole injection layer and further layers arranged between the hole injection layer and the cathode layer.

The operating voltage U is measured in Volt.

In the context of the present specification the term "essentially non-emissive" or "non-emissive" means that the contribution of the compound of formula (I) or the hole injection layer comprising a compound of formula (I), to the visible emission spectrum from an electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

In the context of the present invention, the term "sublimation " may refer to a transfer from solid state to gas phase or from liquid state to gas phase.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The term "HOMO level" is understood to mean the highest occupied molecular orbital and is determined in eV (electron volt).

The term "HOMO level further away from vacuum level" is understood to mean that the absolute value of the HOMO level is higher than the absolute value of the HOMO level of the reference compound. For example, the term "further away from vacuum level than the HOMO level of N2,N2,N2',N2',N7,N7,N7',N7'-octakis(4-methoxyphenyl)-9,9'-spirobi[fluorene]-2,2',7,7'-tetraamine is understood to mean that the absolute value of the HOMO level of the matrix compound of the hole injection layer is higher than the HOMO level of N2,N2,N2',N2', N7,N7, N7',N7'-octakis(4-methoxyphenyl)-9,9'-spirobi[fluorene]-2,2',7,7'-tetraamine.

The term "absolute value" is understood to mean the value without the "- "symbol. According to one embodiment of the present invention, the HOMO level of the matrix compound of the hole injection layer may be calculated by quantum mechanical methods.

### Advantageous Effects

Surprisingly, it was found that the electronic device according to the invention solves the problem underlying the present invention by enabling electronic devices, such as organic light-emitting diodes, in various aspects superior over the electronic devices known in the art, in particular with respect to operating voltage.

Additionally, it was found that the problem underlying the present invention may be solved by providing compounds which may be suitable for deposition through vacuum thermal evaporation under conditions suitable for mass production. In particular, the rate onset temperature of the compound of formula (I) of the present invention may be in a range suitable for mass production.

The compound of Formula (I) is non-emissive. In the context of the present specification the term "essentially non-emissive" or "non-emissive" means that the contribution of the compound of formula (I) to the visible emission spectrum from an electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

### M of the compound of Formula (I)

The term "M" represents a metal. According to one embodiment, the metal M may be selected from alkali, alkaline earth, transition, rare earth metal or group III to V metal, preferably the metal M is selected from transition or group III to V metal; preferably the metal M is selected from Li(I), Na(I), K(I), Cs(I), Mg(II), Ca(II), Sr(II), Ba(II), Sc(III), Y(III), Ti(IV), V(III-V), Cr(III-VI), Mn(II), Mn(III), Fe(II), Fe(III), Co(II), Co(III), Ni(II), Cu(I), Cu(II), Zn(II), Ag(I), Au(I), Au(III), Al(III), Ga(III), In(III), Sn(II), Sn(IV), or Pb(II); preferably M is selected from Cu(II), Fe(III), Co(III), Mn(III), Ir(III), Bi(III); and more preferred M is selected from Fe(III) and Cu(II). Elements of groups IV-XI are named transition metals.

### Ligand L of formula (I)

The term "L" represents a charge-neutral ligand, which coordinates to the metal M. According to one embodiment L is selected from the group comprising H₂O, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (II); 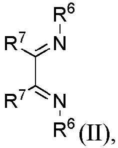 wherein
- R⁶ and R⁷: are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

According to one embodiment, wherein the ligand L in compound of Formula (I) may be selected from a group comprising:
- at least three carbon atoms, alternatively at least four carbon atoms, and/or
- at least two oxygen atoms or one oxygen and one nitrogen atom, two to four oxygen atoms, two to four oxygen atoms and zero to two nitrogen atoms, and/or
- at least one or more groups selected from halogen, F, CN, substituted or unsubstituted C₁ to C₆ alkyl, substituted or unsubstituted C₁ to C₆ alkoxy, alternatively two or more groups selected from halogen, F, CN, substituted or unsubstituted C₁ to C₆ alkyl, substituted or unsubstituted C₁ to C₆ alkoxy, at least one or more groups selected from halogen, F, CN, substituted C₁ to C₆ alkyl, substituted C₁ to C₆ alkoxy, alternatively two or more groups selected from halogen, F, CN, perfluorinated C₁ to C₆ alkyl, perfluorinated C₁ to C₆ alkoxy, one or more groups selected from substituted or unsubstituted C₁ to C₆ alkyl, substituted or unsubstituted C₆ to C₁₂ aryl, and/or substituted or unsubstituted C₃ to C₁₂ heteroaryl,
   wherein the substituents are selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR³, COOR³, halogen, F or CN;
   wherein R³ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

### The term "n"

The term "n" is an integer selected from 1 to 4, which corresponds to the oxidation number of M. According to one embodiment "n" is an integer selected from 1, 2 and 3, which corresponds to the oxidation number of M. According to one embodiment "n" is an integer selected from 1 or 2. According to another embodiment "n" is an integer selected from 1 or 3. According to another embodiment "n" is an integer selected from 2 or 3.

### The term "m"

The term "m" is an integer selected from 0 to 2, which corresponds to the oxidation number of M. According to one embodiment "m" is an integer selected from 0 or 1. According to another embodiment "m" is an integer selected from 1 or 2. According to another embodiment "m" is an integer selected from 0 or 2.

### Embodiments

The compound represented by Formula (I) can be also named metal complex or metal acetylacetonate complex.

According to one embodiment, the metal complex of Formula (I) may have a molecular weight Mw of ≥ 287 g/mol and ≤ 2000 g/mol, preferably a molecular weight Mw of ≥ 400 g/mol and ≤ 1500 g/mol, further preferred a molecular weight Mw of ≥ 580 g/mol and ≤ 1500 g/mol, in addition preferred a molecular weight Mw of ≥ 580 g/mol and ≤ 1400 g/mol.

According to one embodiment the compound is represent by Formula I: 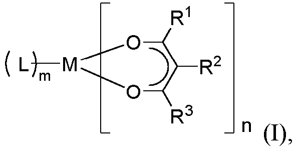 wherein
M is a metal;
L is a charge-neutral ligand, which coordinates to the metal M;
n is an integer selected from 1 to 4, which corresponds to the oxidation number of M;
m is an integer selected from 0 to 2;
R¹ and R³ are independently selected from H, D, substituted or unsubstituted C₁ to C₁₂ alkyl, substituted or unsubstituted C₁ to C₁₂ alkoxy, substituted or unsubstituted C₆ to C₂₄ aryl, and substituted or unsubstituted C₂ to C₂₄ heteroaryl group, wherein the at least one substituent is selected from halogen, F, Cl, CN, substituted or unsubstituted Ci to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein
   the substituents are selected from halogen, F, Cl, CN, C₁ to C₆ alkyl, CF₃, OCH₃ and OCF₃;
R² is independently selected from substituted or unsubstituted C₁ to C₁₂ alkyl, substituted or unsubstituted C₁ to C₁₂ alkoxy, substituted or unsubstituted C₆ to C₂₄ aryl, and substituted or unsubstituted C₂ to C₂₄ heteroaryl group, wherein
   the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy substituted or unsubstituted C₆ to C₁₈ aryl, and substituted or unsubstituted C₂ to C₁₈ heteroaryl, wherein
   the substituents are selected from halogen, F, Cl, CN, C₁ to C₆ alkyl, CF₃, OCH₃ and OCF₃;
wherein at least one R¹, R² and/or R³ is selected from a substituted C₂ to C₂₄ heteroaryl group, wherein at least one substituent is selected from halogen, F, Cl, CN, partially or fully fluorinated C₁ to C₆ alkyl, partially or fully fluorinated C₁ to C₆ alkoxy.

According to one embodiment the compound is represent by Formula I: 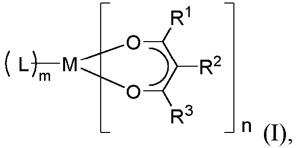 wherein
M is a metal;
L is a charge-neutral ligand, which coordinates to the metal M;
n is an integer selected from 1 to 4, which corresponds to the oxidation number of M;
m is an integer selected from 0 to 2;
R¹ and R³ are independently selected from substituted or unsubstituted C₁ to C₁₂ alkyl, substituted or unsubstituted C₁ to C₁₂ alkoxy, substituted or unsubstituted C₆ to C₂₄ aryl, and substituted or unsubstituted C₂ to C₂₄ heteroaryl group, wherein the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein
   the substituents are selected from halogen, F, Cl, CN, C₁ to C₆ alkyl, CF₃, OCH₃ and OCF₃;
R² is independently selected from H, D, substituted or unsubstituted C₁ to C₁₂ alkyl, substituted or unsubstituted C₁ to C₁₂ alkoxy, substituted or unsubstituted C₆ to C₂₄ aryl, and substituted or unsubstituted C₂ to C₂₄ heteroaryl group, wherein
   the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein the substituents are selected from halogen, F, Cl, CN, C₁ to C₆ alkyl, CF₃, OCH₃ and OCF₃;
wherein one of R¹, R² and R³ is selected from a substituted C₂ to C₂₄ heteroaryl group, wherein at least one substituent is selected from halogen, F, Cl, CN, CF₃, partially or fully fluorinated Ci to C₆ alkyl, partially or fully fluorinated C₁ to C₆ alkoxy.

According to one embodiment the compound is represent by Formula I: 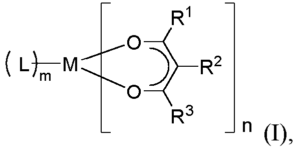 wherein
M is a metal;
L is a charge-neutral ligand, which coordinates to the metal M;
n is an integer selected from 1 to 4, which corresponds to the oxidation number of M;
m is an integer selected from 0 to 2;
R¹, R² are independently selected from H, D, substituted or unsubstituted C₁ to C₁₂ alkyl, substituted or unsubstituted C₁ to C₁₂ alkoxy, substituted or unsubstituted C₆ to C₂₄ aryl, and substituted or unsubstituted C₂ to C₂₄ heteroaryl group, wherein the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein
   the substituents are selected from halogen, F, Cl, CN, C₁ to C₆ alkyl, CF₃, OCH₃ and OCF₃;
   wherein R¹ or R² is selected from a substituted C₂ to C₂₄ heteroaryl group, wherein at least one substituent is selected from halogen, F, Cl, CN, CF₃; and
R³ is H, D, substituted or unsubstituted C₁ to C₁₂ alkyl, wherein the substituents are selected from halogen, F, Cl, CN, CF₃.

According to one embodiment the compound is represent by Formula I: 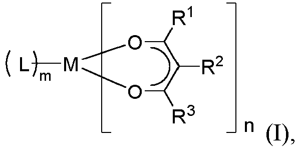 wherein
M is a metal;
L is a charge-neutral ligand, which coordinates to the metal M;
n is an integer selected from 1 to 4, which corresponds to the oxidation number of M;
m is an integer selected from 0 to 2;
R¹, R² are independently selected from H, D, substituted or unsubstituted C₁ to C₁₂ alkyl, substituted or unsubstituted C₁ to C₁₂ alkoxy, substituted or unsubstituted C₆ to C₂₄ aryl, and substituted or unsubstituted C₂ to C₂₄ heteroaryl group, wherein
   the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein the substituents are selected from halogen, F, Cl, CN, C₁ to C₆ alkyl, CF₃, OCH₃ and OCF₃;
   wherein R¹ or R² is selected from a substituted C₂ to C₂₄ heteroaryl group, preferably a 6 member heteroaryl ring with 1, 2 or 3 N atoms and the remaining atoms are C, wherein at least one, two, three or four of the remaining C-atoms of the heteroaryl ring are substituent with a substituent independently selected from the group halogen, F, Cl, CN, CF₃, preferably F, CN, CF₃; and
R³ is H, D, CH₃, CF₃, CN, preferably CH₃ or CF₃.

According to one embodiment the compound is represent by Formula I: 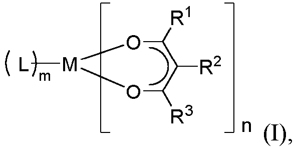 wherein
M is a metal;
L is a charge-neutral ligand, which coordinates to the metal M;
n is an integer selected from 1 to 4, which corresponds to the oxidation number of M;
m is an integer selected from 0 to 2;
R¹, R² are independently selected from H, D, substituted or unsubstituted C₁ to C₁₂ alkyl, substituted or unsubstituted C₂ to C₂₄ heteroaryl group, wherein
   the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein
   the substituents are selected from halogen, F, Cl, CN, C₁ to C₆ alkyl, CF₃, OCH₃ and OCF₃;
   wherein R¹ or R² is selected from a 6 member heteroaryl ring with 1, 2 or 3 N atoms and the remaining atoms are C, wherein at least one, two, three or four of the remaining C-atoms of the heteroaryl ring are substituent with a substituent individually selected from the group halogen, F, Cl, CN, CF₃, preferably F, CN, CF₃; and
R³ is CH₃, CF₃, CN preferably CH₃ or CF₃.

According to one embodiment the compound is represented by Formula I: 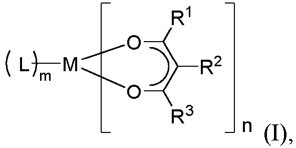 wherein
M is a metal;
L is a charge-neutral ligand, which coordinates to the metal M;
n is an integer selected from 1 to 4, which corresponds to the oxidation number of M;
m is an integer selected from 0 to 2;
R¹, R² and R³ are independently selected from H, D, substituted or unsubstituted C₁ to C₁₂ alkyl, substituted or unsubstituted C₆ to C₂₄ aryl, and substituted or unsubstituted C₂ to C₂₄ heteroaryl group, wherein
   the at least one substituent is selected from halogen, F, Cl, CN, substituted or unsubstituted C₁ to C₆ alkyl, partially or fully fluorinated C₁ to C₆ alkyl, substituted or unsubstituted C₆ to C₁₈ aryl, and substituted or unsubstituted C₂ to C₁₈ heteroaryl, wherein
   the substituents are selected from halogen, F, Cl, CN, C₁ to C₆ alkyl, CF₃, and OCF₃;
wherein
   at least one R¹, R² and/or R³ is selected from a substituted C₂ to C₂₄ heteroaryl group, wherein at least one substituent is selected from halogen, F, Cl, CN, partially or fully fluorinated C₁ to C₆ alkyl.

According to one embodiment, wherein R¹, R² and R³ are independently selected from H, D, substituted or unsubstituted C₁ to C₁₂ alkyl, substituted or unsubstituted C₁ to C₁₂ alkoxy, substituted or unsubstituted C₆ to C₂₄ aryl, and substituted or unsubstituted C₂ to C₂₄ heteroaryl group, wherein the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein the substituents are selected from halogen, F, Cl, CN, C₁ to C₆ alkyl, CF₃, OCH₃ and OCF₃.

According to one embodiment, wherein R¹, R² and R³ are independently selected from H, D, substituted or unsubstituted C₁ alkyl, substituted C₆ to C₂₄ aryl, and substituted C₂ to C₂₄ heteroaryl group, wherein the at least one substituent is selected from halogen, F, Cl, CN, CF₃.

According to one embodiment, wherein at least one R¹, R² or R³ is selected from a substituted C₂ to C₂₄ heteroaryl group, wherein
- the substituted heteroaryl group comprises at least one six-membered ring; and/or
- the substituted heteroaryl group comprises at least 1 to 3 N atoms, preferably 1 to 2 N atoms, also preferred 1 N atom; and/or
- the heteroaryl group of the substituted heteroaryl group is a six-membered ring, comprise 1, 2 or 3 hetero atoms, wherein preferably the hetero atom is N.

According to one embodiment, wherein at least one R¹, R² or R³ is selected from a substituted C₂ to C₂₄ heteroaryl group, wherein the heteroaryl group is selected from pyridyl, pyrimidinyl pyrazinyl, or triazinyl.

According to one embodiment, wherein at least one R¹, R² or R³ is selected from a substituted C₂ to C₂₄ heteroaryl group, wherein the at least one substituent of the substituted heteroaryl group is selected from the group comprising halogen, F, Cl, CN, partially or fully fluorinated C₁ to C₆ alkyl, partially or fully fluorinated C₁ to C₆ alkoxy; preferably from the group comprising halogen, F, Cl, CN, partially or fully fluorinated C₁ to C₆ alkyl, partially or fully fluorinated C₁ to C₆ alkoxy; further preferred from the group comprising halogen, F, Cl, CN, partially or fully fluorinated Ci to C₄ alkyl, partially or fully fluorinated C₁ to C4 alkoxy; and more preferred from the group comprising F, CN, partially or fully fluorinated C₁ to C₆ alkyl; also preferred F, CN, partially or fully fluorinated C₁ to C₆ alkyl, also preferred halogen, F, Cl, CN, partially or fully fluorinated C₁ to C₄ alkyl; and more preferred from the group comprising at least one CN, at least one CF₃ group, and/or at least two F atoms.

According to one embodiment, wherein one R¹, R² or R³ is selected from a substituted C₂ to C₂₄ heteroaryl group, wherein at least one substituent is selected from halogen, F, Cl, CN, partially or fully fluorinated C₁ to C₆ alkyl, partially or fully fluorinated C₁ to C₆ alkoxy., and one R¹, R² or R³ is selected from substituted or unsubstituted C₁ to C₁₂ alkyl or substituted or unsubstituted C₁ to C₁₂ alkoxy, wherein the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy; and one R¹, R² or R³ is selected from H, D, substituted or unsubstituted C₁ to C₁₂ alkyl, substituted or unsubstituted C₁ to C₁₂ alkoxy, wherein the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy; wherein
the substituents are selected from halogen, F, Cl, CN, C₁ to C₆ alkyl, CF₃, OCH₃ and OCF₃.

According to one embodiment, wherein R¹ or R² is selected from a substituted C₂ to C₂₄ heteroaryl group and the remaining R¹, R² and R³ are selected from H, substituted or unsubstituted C₁ to C₁₂ alkyl, substituted or unsubstituted C₁ to C₁₂ alkoxy, wherein the at least one substituent is selected from halogen, F, Cl, CN, C₁ to C₆ alkyl, C₁ to C₆ alkoxy; wherein R¹ and R³ are not H.

According to one embodiment, wherein R¹, R² are selected from unsubstituted C₁ to C₁₂ alkyl, preferably CH₃, or a substituted 6-member heteroaryl ring with 1, 2 or 3 N atoms and the remaining atoms are C, wherein the at least one substituent is selected from F, CN, CF₃; wherein R¹ or R² is selected from a 6 member heteroaryl ring with 1, 2 or 3 N atoms and the remaining atoms are C, wherein at least one, two, three or four of the remaining C-atoms of the heteroaryl ring are substituent with a substituent individually selected from the group halogen, F, Cl, CN, CF₃, preferably F, CN, CF₃; and R³ is CH₃, CF₃, CN preferably CH₃ or CF₃.

According to one embodiment, wherein R¹, R² are selected from unsubstituted C₁ to C₁₂ alkyl, preferably CH₃, or a substituted 6-member heteroaryl ring with 1, 2 or 3 N atoms and the remaining atoms are C, wherein the at least one substituent is selected from F, CN, CF₃; wherein R¹ or R² is selected from a 6 member heteroaryl ring with 1, 2 or 3 N atoms and the remaining atoms are C, wherein at least one of the remaining C-atoms of the heteroaryl ring are substituent with a substituent individually selected from the group halogen, F, Cl, CN, CF₃, preferably F, CN, CF₃; and R³ is CH₃, CF₃, CN preferably CH₃ or CF₃.

According to one embodiment, wherein R¹, R² are selected from unsubstituted C₁ to C₁₂ alkyl, preferably CH₃, or a substituted 6-member heteroaryl ring with 1, 2 or 3 N atoms and the remaining atoms are C, wherein the at least one substituent is selected from F, CN, CF₃; wherein R¹ or R² is selected from a 6 member heteroaryl ring with 1, 2 or 3 N atoms and the remaining atoms are C, wherein at least two of the remaining C-atoms of the heteroaryl ring are substituent with a substituent individually selected from the group halogen, F, Cl, CN, CF₃, preferably F, CN, CF₃; and R³ is CH₃, CF₃, CN preferably CH₃ or CF₃.

According to one embodiment, wherein R¹, R² are selected from unsubstituted C₁ to C₁₂ alkyl, preferably CH₃, or a substituted 6-member heteroaryl ring with 1, 2 or 3 N atoms and the remaining atoms are C, wherein the at least one substituent is selected from F, CN, CF₃; wherein R¹ or R² is selected from a 6 member heteroaryl ring with 1, 2 or 3 N atoms and the remaining atoms are C, wherein at least three of the remaining C-atoms of the heteroaryl ring are substituent with a substituent individually selected from the group halogen, F, Cl, CN, CF₃, preferably F, CN, CF₃; and R³ is CH₃, CF₃, CN preferably CH₃ or CF₃.

According to one embodiment, wherein R¹, R² are selected from unsubstituted C₁ to C₁₂ alkyl, preferably CH₃, or a substituted 6-member heteroaryl ring with 1, 2 or 3 N atoms and the remaining atoms are C, wherein the at least one substituent is selected from F, CN, CF₃; wherein R¹ or R² is selected from a 6 member heteroaryl ring with 1 or 2 N atoms and the remaining atoms are C, wherein at least four of the remaining C-atoms of the heteroaryl ring are substituent with a substituent individually selected from the group halogen, F, Cl, CN, CF₃, preferably F, CN, CF₃; and R³ is CH₃, CF₃, CN preferably CH₃ or CF₃.

According to one embodiment, the at least one substituent on the C₂ to C₂₄ heteroaryl group is selected from halogen, F, Cl, CN, partially or fully fluorinated C₁ to C₆ alkyl, partially or fully fluorinated C₁ to C₆ alkoxy; more preferred halogen, F, Cl, CN, partially or fully fluorinated C₁ to C₆ alkyl, partially or fully fluorinated C₁ to C₆ alkoxy, also preferred halogen, F, Cl, CN, partially or fully fluorinated C₁ to C₄ alkyl, partially or fully fluorinated C₁ to C₄ alkoxy.

According to one embodiment, the at least one substituent on the C₂ to C₂₄ heteroaryl group is selected from F, CN, partially or fully fluorinated C₁ to C₆ alkyl; more preferred F, CN, partially or fully fluorinated C₁ to C₆ alkyl, also preferred halogen, F, Cl, CN, partially or fully fluorinated C₁ to C₄ alkyl.

According to one embodiment, the at least one substituent on the C₂ to C₂₄ heteroaryl group is selected from at least one CN or CF₃ group or at least two F atoms.

According to one embodiment, wherein R¹, R², R³ may be not selected from a substituted or unsubstituted C₆ to C₂₄ aryl group or a substituted or unsubstituted C₆ to C₁₈.

According to one embodiment, wherein R¹, R², R³ may be not selected from a substituted or unsubstituted aryl group.

According to one embodiment, wherein Formula I may not comprise a substituted or unsubstituted C₆ to C₂₄ aryl group or a substituted or unsubstituted C₆ to C₁₈.

According to one embodiment, wherein Formula I may not comprises a substituted or unsubstituted aryl group.

According to one embodiment, wherein at least one substituted C₂ to C₂₄ heteroaryl group of R¹, R² or R³ is selected from the following Formulas D1 to D29: 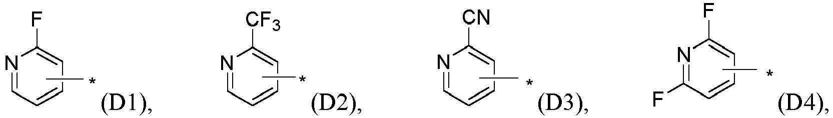 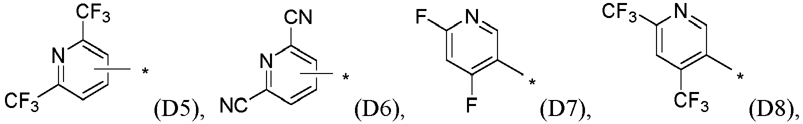 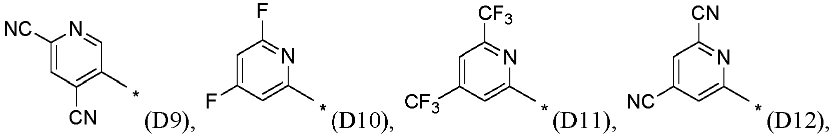 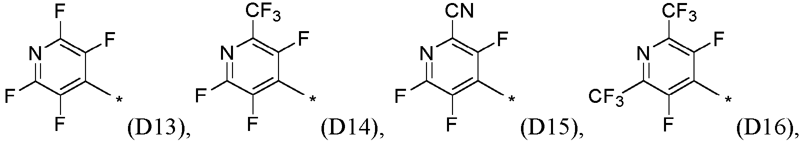 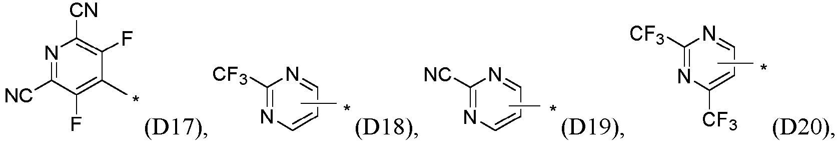 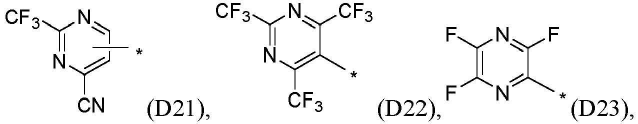 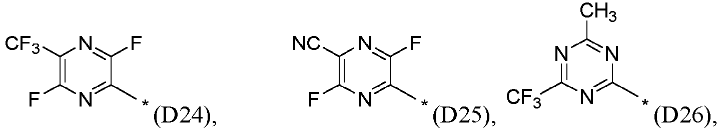 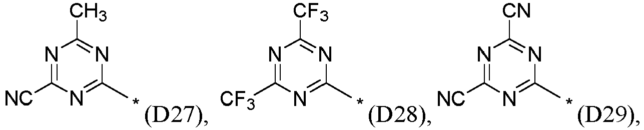 wherein the "*" denotes the binding position.

According to one embodiment, wherein the compound represented by Formula I is selected from the following Formulas E1 to E28:

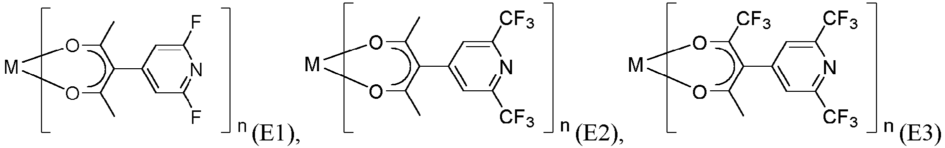

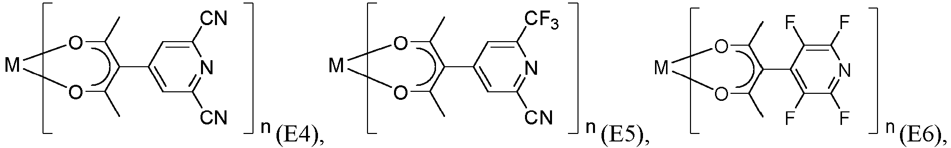

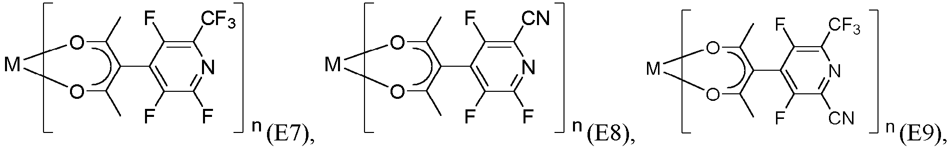

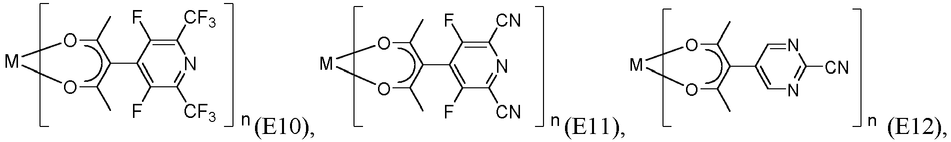

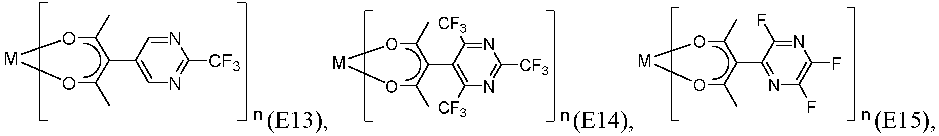

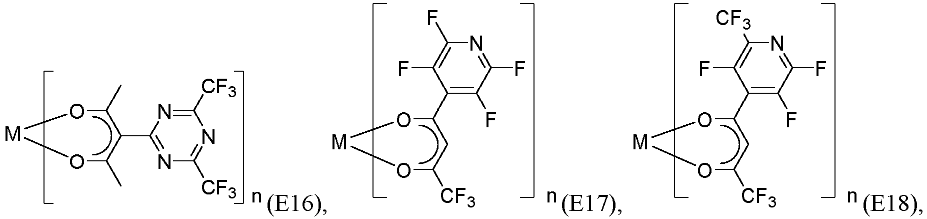

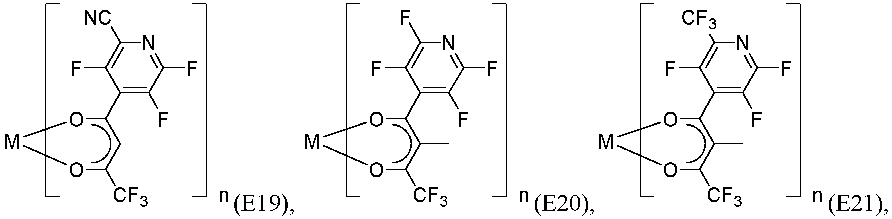

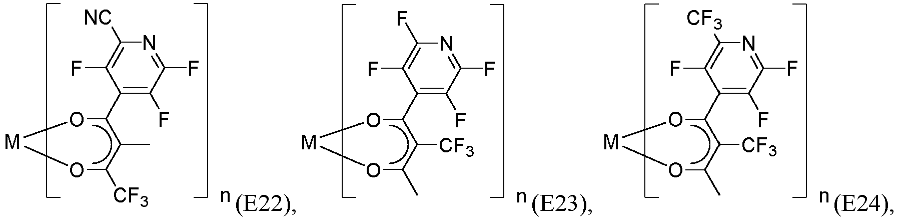

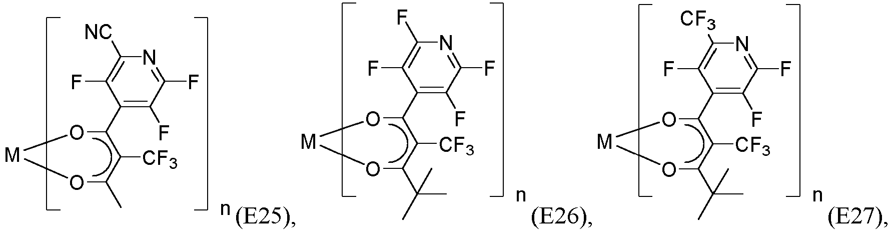

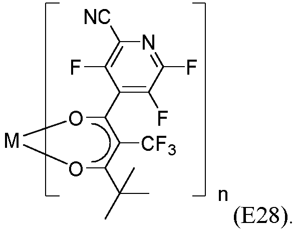

According to one embodiment, wherein the compound represented by Formula I is selected from the following Formulas G1 to G48:

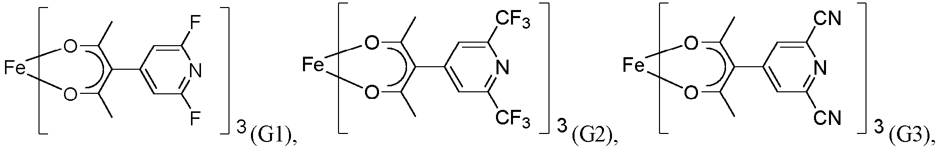

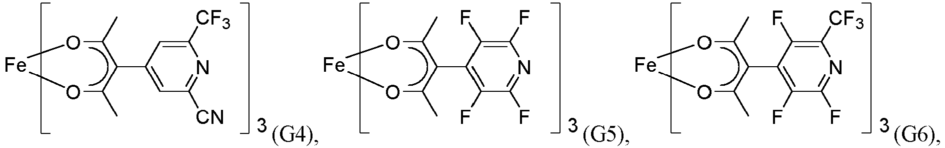

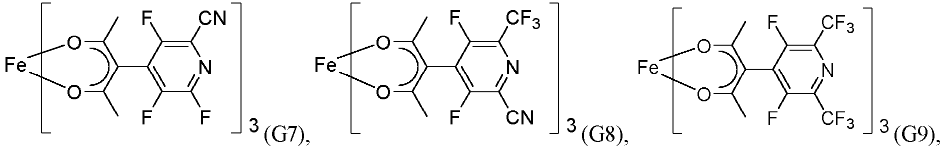

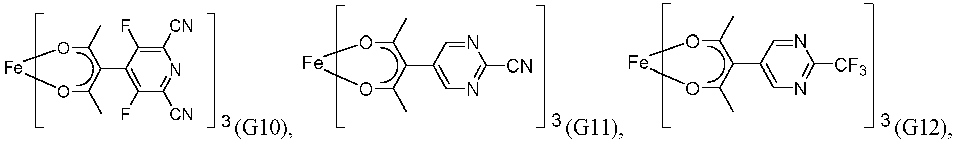

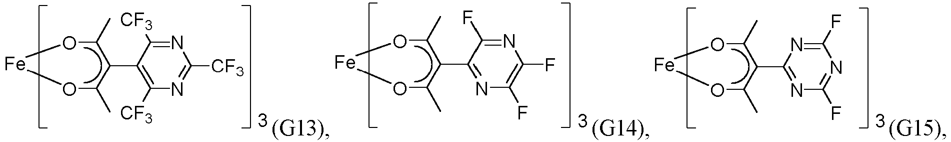

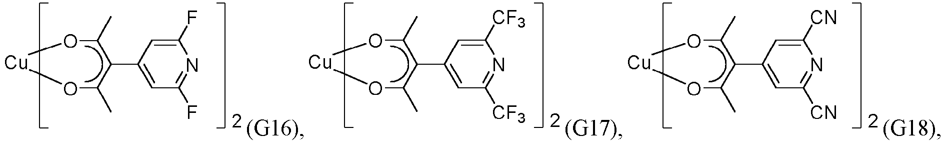

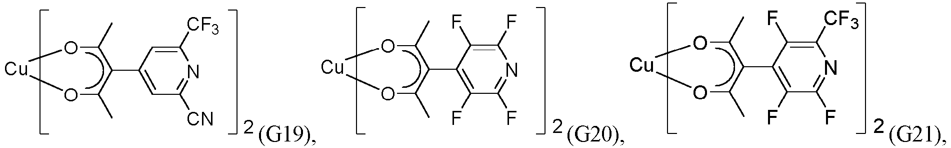

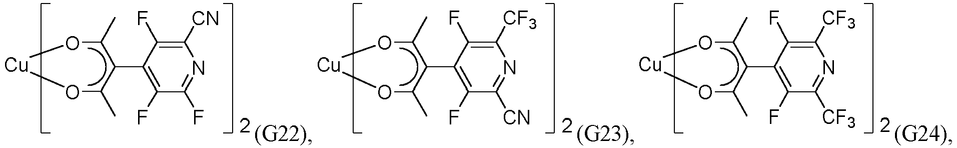

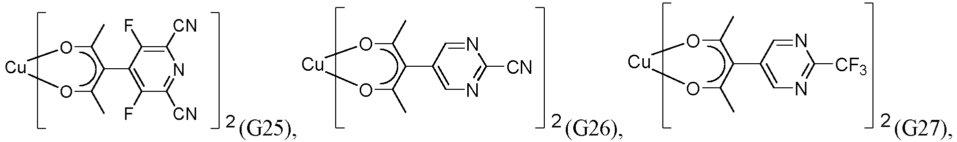

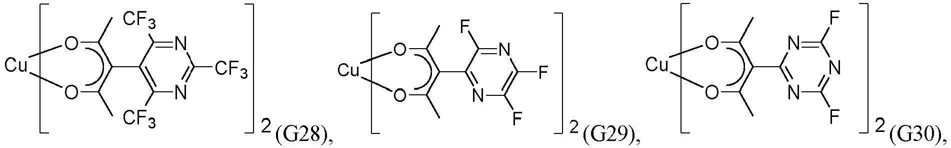

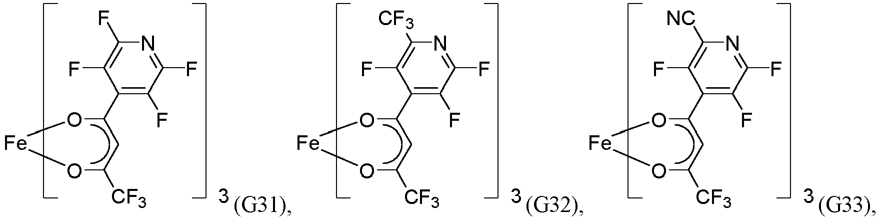

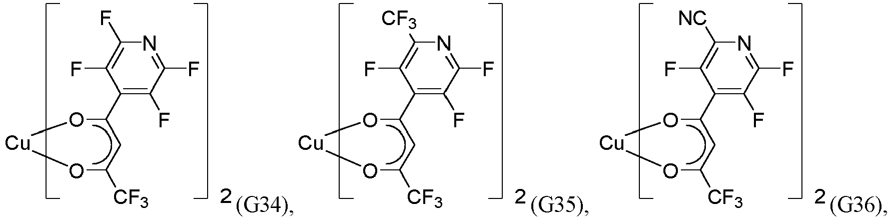

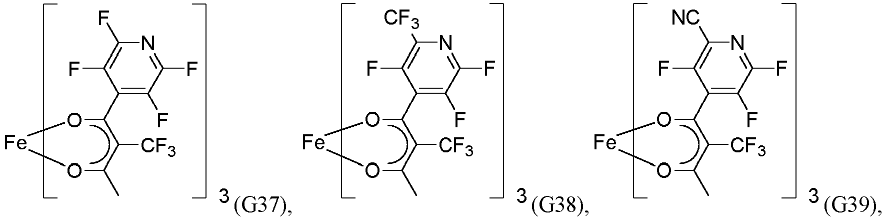

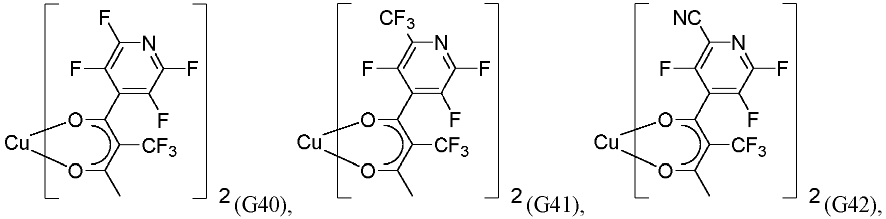

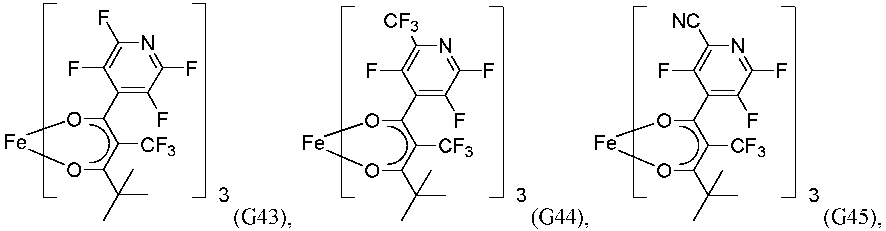

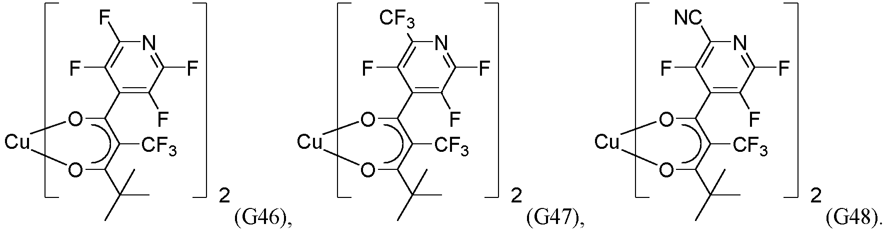

### Substantially covalent matrix compound

The substantially covalent matrix compound, also named matrix compound, may be an organic aromatic matrix compounds, which comprises organic aromatic covalent bonded carbon atoms. The substantially covalent matrix compound may be an organic compound, consisting substantially from covalently bound C, H, O, N, S, which may optionally comprise also covalently bound B, P or Si. The substantially covalent matrix compound may be an organic aromatic covalent bonded compound, which is free of metal atoms, and the majority of its skeletal atoms may be selected from C, O, S, N and preferably from C, O and N, wherein the majority of atoms are C-atoms. Alternatively, the covalent matrix compound is free of metal atoms and majority of its skeletal atoms may be selected from C and N, preferably the covalent matrix compound is free of metal atoms and majority of its skeletal atoms may be selected from C and the minority of its skeletal atoms may be N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

In one embodiment of the present invention, the substantially covalent matrix compound may be free of alkoxy groups.

Preferably, the substantially covalent matrix compound comprises at least one arylamine moiety, alternatively a diarylamine moiety, alternatively a triarylamine moiety.

Preferably, the substantially covalent matrix compound is free of TPD or NPB.

Preferably, the matrix compound of the hole injection layer is free of metals and/or ionic bonds.

### Compound of formula (III) or a compound of formula (IV)

According to another aspect of the present invention, the substantially covalent matrix compound may comprises at least one arylamine compound, diarylamine compound, triarylamine compound, a compound of formula (III) or a compound of formula (IV): 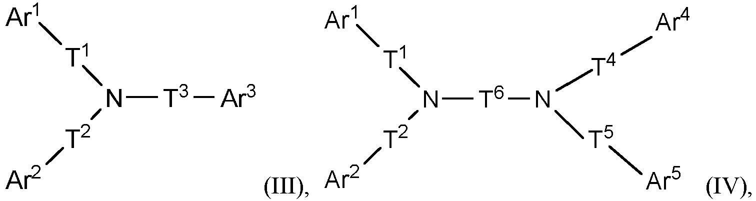 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,fJazepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
   wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment of the electronic device, wherein the substantially covalent matrix compound comprises a compound of formula (III) or formula (IV): 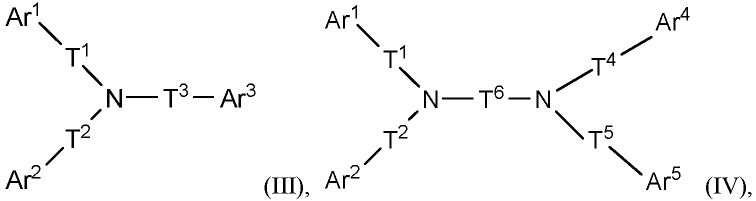 wherein
T¹, T², T³, T⁴ and T⁵ may be independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ may be independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,fJazepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein the substituents of Ar¹, Ar², Ar³, Ar⁴ and Ar⁵ are selected the same or different from the group comprising H, straight-chain alkyl having 1 to 20 carbon atoms, branched alkyl having 1 to 20 carbon atoms, cyclic alkyl having 3 to 20 carbon atoms, alkenyl or alkynyl groups having 2 to 20 carbon atoms, alkoxy groups having 1 to 20 carbon atoms, C₆ to C₁₈ aryl, C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle.

Preferably, the substituents of Ar¹, Ar², Ar³, Ar⁴ and Ar⁵ are selected the same or different from the group comprising H, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, alkoxy groups having 1 to 6 carbon atoms, C₆ to C₁₈ aryl, C₃ to C₁₈ heteroaryl, a fused ring system comprising 2 to 4 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle; more preferred the substituents are selected the same or different from the group consisting of H, straight-chain alkyl having 1 to 4 carbon atoms, branched alkyl having 1 to 4 carbon atoms, cyclic alkyl having 3 to 4 carbon atoms and/or phenyl. Thereby, the compound of formula (III) or (IV) may have a rate onset temperature suitable for mass production.

According to an embodiment of the electronic device, wherein the substantially covalent matrix compound comprises a compound of formula (III) or formula (IV): 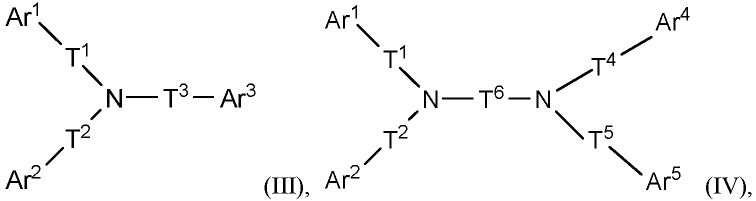 wherein
T¹, T², T³, T⁴ and T⁵ may be independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,fJazepine, unsubstituted 9,9'-spirobi[fluorene], unsubstituted spiro[fluorene-9,9'-xanthene], or a unsubstituted aromatic fused ring system comprising at least three unsubstituted aromatic rings selected from the group comprising unsubstituted non-hetero, unsubstituted hetero 5-member rings, unsubstituted 6-member rings and/or unsubstituted 7-member rings, unsubstituted fluorene, or a fused ring system comprising 2 to 6 unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle.

According to an embodiment of the electronic device, wherein the substantially covalent matrix compound comprises a compound of formula (III) or formula (IV): 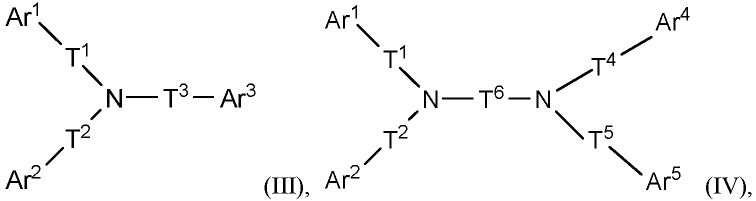 wherein
T¹, T², T³, T⁴ and T⁵ may be independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,fJazepine, unsubstituted 9,9'-spirobi[fluorene], unsubstituted spiro[fluorene-9,9'-xanthene].

Thereby, the compound of formula (III) or (IV) may have a rate onset temperature suitable for mass production.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from B1 to B16: 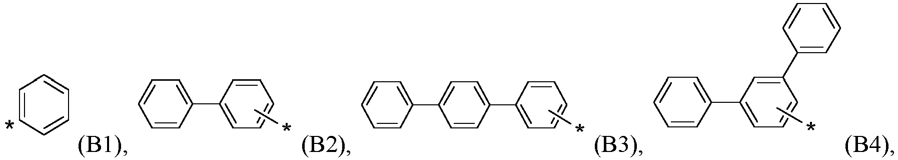 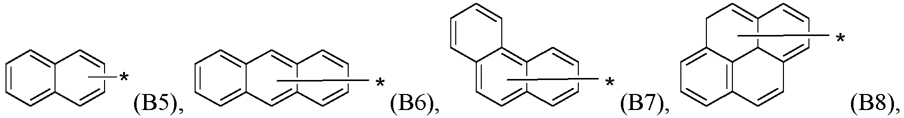 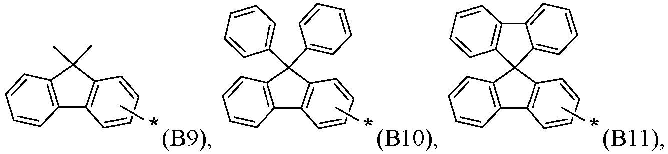 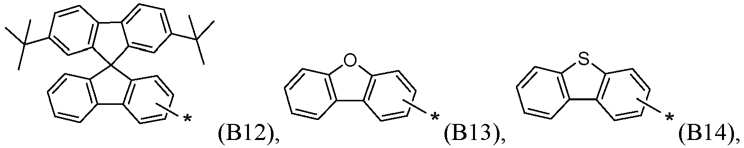 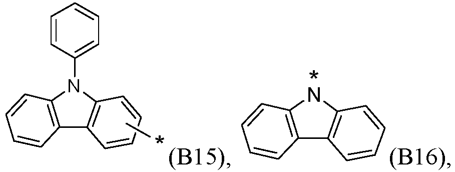 wherein the asterix "*" denotes the binding position.

According to an embodiment, wherein Ar¹, Ar², Ar³, Ar⁴ and Ar⁵ may be independently selected from B1 to B15; alternatively selected from B1 to B10 and B13 to B15.

According to an embodiment, wherein Ar¹, Ar², Ar³, Ar⁴ and Ar⁵ may be independently selected from the group consisting of B1, B2, B5, B7, B9, B10, B13 to B16.

The rate onset temperature may be in a range particularly suited to mass production, when Ar¹, Ar², Ar³, Ar⁴ and Ar⁵ are selected in this range.

The "matrix compound of formula (III) or formula (IV) " may be also referred to as "hole transport compound".

According to one embodiment the compound of formula (III) or formula (IV) may comprises at least ≥ 1 to ≤ 6 substituted or unsubstituted aromatic fused ring systems comprising heteroaromatic rings.

According to one embodiment the compound of formula (III) or formula (IV) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings.

According to one embodiment the compound of formula (III) or formula (IV) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings, and at least ≥ 1 to ≤ 3 substituted or unsubstituted unsaturated 5- to 7-member ring of a heterocycle, further preferred 3 or 4 substituted or unsubstituted aromatic fused ring systems comprising heteroaromatic rings and optional at least ≥ 1 to ≤ 3 substituted or unsubstituted unsaturated 5- to 7-member ring of a heterocycle, and additional preferred wherein the aromatic fused ring systems comprising heteroaromatic rings are unsubstituted and optional at least ≥ 1 to ≤ 3 unsubstituted unsaturated 5- to 7-member ring of a heterocycle.

According to one embodiment the compound of formula (III) or formula (IV) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems.

According to one embodiment the compound of formula (III) or formula (IV) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings.

According to one embodiment the compound of formula (III) or formula (IV) may comprises at least ≥ 1 to ≤ 3 or 2 substituted or unsubstituted unsaturated 5- to 7-member ring of a heterocycle.

According to one embodiment the compound of formula (III) or formula (IV) may comprises at least ≥ 1 to ≤ 3 or 2 substituted or unsubstituted unsaturated 7-member ring of a heterocycle.

According to one embodiment substituted or unsubstituted aromatic fused ring systems of the compound of formula (III) or formula (IV) may comprises at least ≥ 1 to ≤ 3 or 2 substituted or unsubstituted unsaturated 5- to 7-member ring of a heterocycle.

According to one embodiment the substituted or unsubstituted aromatic fused ring systems of the matrix compound of formula (III) or formula (IV) may comprises at least ≥ 1 to ≤ 3 or 2 substituted or unsubstituted unsaturated 7-member ring of a heterocycle.

According to one embodiment the compound of formula (III) or formula (IV) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises substituted or unsubstituted unsaturated 5- to 7-member ring of a heterocycle.

According to one embodiment the compound of formula (III) or formula (IV) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises substituted or unsubstituted unsaturated 5- to 7-member ring of a heterocycle.

According to one embodiment the compound of formula (III) or formula (IV) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises at least ≥ 1 to ≤ 3 or 2 substituted or unsubstituted unsaturated 5- to 7-member ring of a heterocycle.

According to one embodiment the compound of formula (III) or formula (IV) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises at least ≥ 1 to ≤ 3 or 2 substituted or unsubstituted unsaturated 5- to 7-member ring of a heterocycle.

According to one embodiment the compound of formula (III) or formula (IV) may comprises:
- a substituted or unsubstituted aromatic fused ring systems with at least ≥ 2 to ≤ 6, preferably ≥ 3 to ≤ 5, or 4 fused aromatic rings selected from the group comprising substituted or unsubstituted non-hetero aromatic rings, substituted or unsubstituted hetero 5-member rings, substituted or unsubstituted 6-member rings and/or substituted or unsubstituted unsaturated 5-to 7- member ring of a heterocycle; or
- an unsubstituted aromatic fused ring systems with at least ≥ 2 to ≤ 6, preferably ≥ 3 to ≤ 5, or 4 fused aromatic rings selected from the group comprising unsubstituted non-hetero aromatic rings, unsubstituted hetero 5-member rings, unsubstituted 6-member rings and/or unsubstituted unsaturated 5- to 7-member ring of a heterocycle.

It should be noted here that the wording "aromatic fused ring system" may include at least one aromatic ring and at least one substituted or unsubstituted unsaturated 5- to 7- member ring. It should be noted here that the substituted or unsubstituted unsaturated 5- to 7- member ring may not be an aromatic ring.

According to one embodiment the compound of formula (III) or formula (IV) may comprises at least at least ≥ 1 to ≤ 6, preferably ≥ 2 to ≤ 5, or further preferred 3 or 4 of the substituted or unsubstituted aromatic fused ring systems with:
- at least one unsaturated 5-member ring, and/or
- at least one unsaturated 6-member ring, and/or
- at least one unsaturated 7-member ring; wherein preferably at least one unsaturated 5- and/or at least one unsaturated 7-member ring comprises at least 1 to 3, preferably 1 hetero-atom.

According to one embodiment the compound of formula (III) or formula (IV) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
wherein the substituted or unsubstituted aromatic fused ring system comprises at least ≥ 1 to ≤ 3 or 2 substituted or unsubstituted unsaturated 5- to 7-member ring of a heterocycle.

According to one embodiment the compound of formula (III) or formula (IV) may comprises :
- at least ≥ 6 to ≤ 12, preferably ≥ 7 to ≤ 11, further preferred ≥ 8 to ≤ 10 or 9 aromatic rings; and/or
- at least ≥ 4 to ≤ 11, preferably ≥ 5 to ≤ 10, further preferred ≥ 6 to ≤ 9 or in addition preferred 7 or 8 non-hetero aromatic rings, preferably the non-hetero aromatic rings are aromatic C₆ rings; and/or
- at least ≥ 1 to ≤ 4, preferably 2 or 3 aromatic 5-member-rings, preferably hetero aromatic 5-member-rings; and/or
- at least 1 or 2 unsaturated 5- to 7-member-ring of a heterocycle, preferably at least 1 or 2 unsaturated 7-member-ring of a heterocycle;
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and/or
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and
      the hole transport compound or the hole transport compound according to formula I comprises at least ≥ 1 to ≤ 4, preferably 2 or 3 aromatic 5-member-rings, preferably hetero aromatic 5-member-rings, and/or
      the hole transport compound or the hole transport compound according to formula (I) comprises at least 1 or 2 unsaturated 5- to 7-member-ring of a heterocycle, preferably at least 1 or 2 unsaturated 7-member-ring of a heterocycle.

According to one embodiment the compound of formula (III) or formula (IV) may comprises a hetero-atom, which may be selected from the group comprising O, S, N, B or P, preferably the hetero-atom may be selected from the group comprising O, S or N.

According to one embodiment the matrix compound of formula (III) or formula (IV) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
wherein the substituted or unsubstituted aromatic fused ring system optional comprises at least ≥ 1 to ≤ 3 or 2 substituted or unsubstituted unsaturated 5- to 7-member ring of a heterocycle; and wherein the substituted or unsubstituted aromatic fused ring system comprises a hetero-atom, which may be selected from the group comprising O, S, N, B, P or Si, preferably the hetero-atom may be selected from the group comprising O, S or N.

According to one embodiment the compound of formula (III) or formula (IV) may be free of hetero-atoms which are not part of an aromatic ring and/or part of an unsaturated 7-member-ring, preferably the hole transport compound or the hole transport compound according to formula (I) may be free on N-atoms except N-atoms which are part of an aromatic ring or are part of an unsaturated 7-member-ring.

According to one embodiment, the substantially covalent matrix compound comprises at least one naphthyl group, carbazole group, dibenzofurane group, dibenzothiophene group and/or substituted fluorenyl group, wherein the substituents are independently selected from methyl, phenyl or fluorenyl.

According to an embodiment of the electronic device, wherein the compound of formula (III) or formula (IV) are selected from K1 to K15:

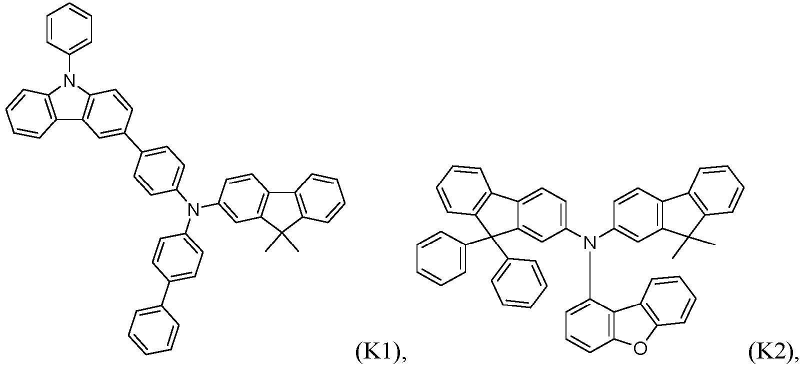

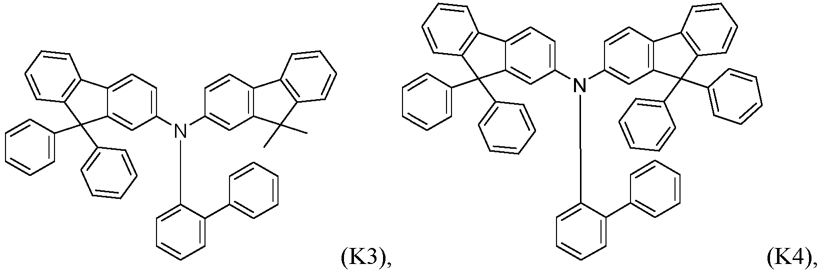

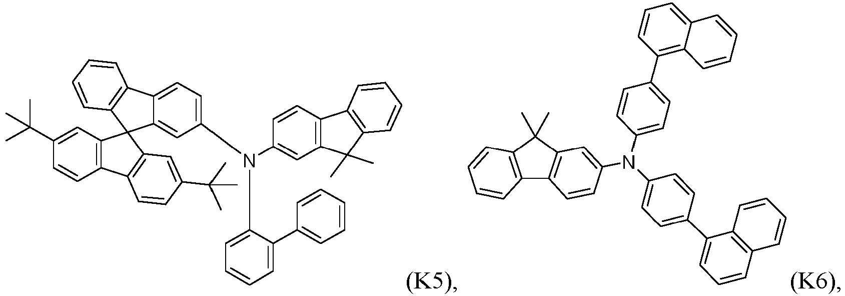

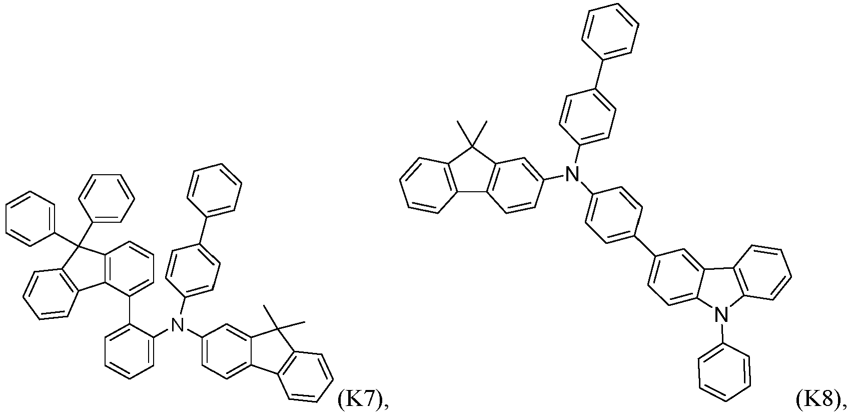

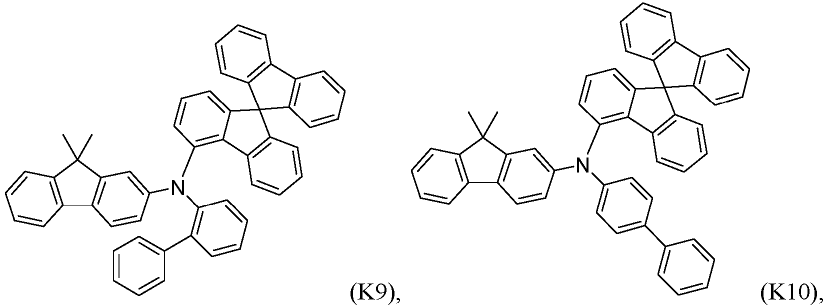

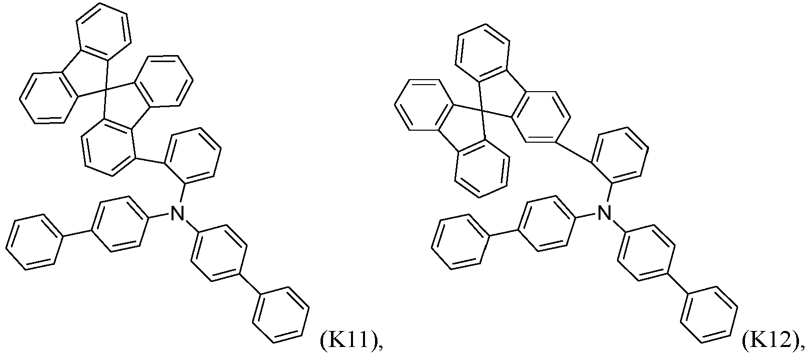

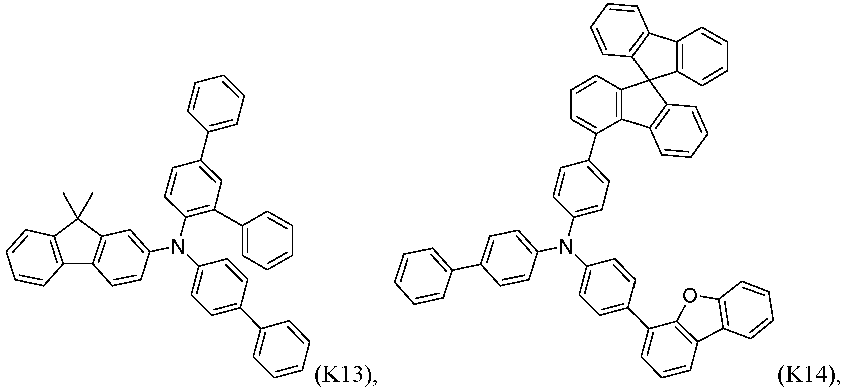

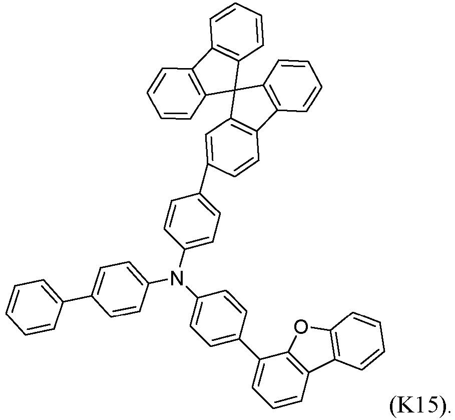

The substantially covalent matrix compound may be free of HTM014, HTM081, HTM163, HTM222, EL-301, HTM226, HTM355, HTM133, HTM334, HTM604 and EL-22T. The abbreviations denote the manufacturers' names, for example, of Merck or Lumtec.

### Semiconductor material

According to another aspect there is provided a semiconductor material comprising at least one compound of Formula I. The semiconductor material may comprises in addition at least one substantially covalent matrix compound.

### Semiconductor layer

According to another aspect, wherein an semiconductor layer comprises at least one compound of Formula I.

According to one embodiment the semiconductor layer comprises at least one compound of Formula I is a hole injection layer.

According to another embodiment the semiconductor layer comprising a semiconductor material containing at least one compound of Formula I.

### Electronic device

According to another embodiment the electronic device comprises a substrate, an anode layer free of sub-layers or an anode layer which may comprise two or more sub-layers, a cathode layer and a hole injection layer, wherein the hole injection layer comprises a compound according to formula (I).

The electronic device may comprise at least one photoactive layer. The at least one photoactive layer may be an emission layer or a light-absorption layer, preferably an emission layer.

According to another embodiment, the electronic device may have the following layer structure, wherein the layers having the following order:
an anode layer, a hole injection layer comprising a substantially covalent matrix compound and a compound of formula (I), a hole transport layer, optional an electron blocking layer, at least a first emission layer, optional a hole blocking layer, an electron transport layer, optional an electron injection layer, and a cathode layer.

According to another aspect, it is provided an electronic device comprising a semiconductor material containing a compound according to Formula I and an semiconductor layer containing a compound according to Formula I. The electronic device can be selected from devices comprising a light emitting device, thin film transistor, a battery, a display device or a photovoltaic cell, and preferably a light emitting device, preferably the electronic device is part of a display device or lighting device.

According to another aspect, it is provided an electronic device comprising at least one organic light emitting device according to any embodiment described throughout this application, preferably, the electronic device comprises the organic light emitting diode in one of embodiments described throughout this application. More preferably, the electronic device is a display device.

According to one embodiment of the present invention, wherein the electronic device may comprise an semiconductor layer comprising a compound of formula (I) and a substantially covalent matrix compound comprising at least one arylamine compound, diarylamine compound, triarylamine compound, wherein in formula (I) M is selected from Li(I), Na(I), K(I), Cs(I), Mg(II), Ca(II), Sr(II), Ba(II), Sc(III), Y(III), Ti(IV), V(III-V), Cr(III-VI), Mn(II), Mn(III), Fe(II), Fe(III), Co(II), Co(III), Ni(II), Cu(I), Cu(II), Zn(II), Ag(I), Au(I), Au(III), Al(III), Ga(III), In(III), Sn(II), Sn(IV), or Pb(II); preferably M is selected from Cu(II), Fe(III), Co(III), Mn(III), Ir(III), Bi(III); and more preferred M is selected from Fe(III) and Cu(II).

### Anode layer

The anode layer, also named anode electrode, may be formed by depositing or sputtering a material that is used to form the anode layer. The material used to form the anode layer may be a high work-function material, so as to facilitate hole injection. The anode layer may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode layer. The anode layer may also be formed using metals, typically silver (Ag), gold (Au), or metal alloys.

The anode layer may comprise two or more anode sub-layers.

According to one embodiment, the anode layer comprises a first anode sub-layer and a second anode sub-layer, wherein the first anode sub-layer is arranged closer to the substrate and the second anode sub-layer is arranged closer to the cathode layer.

According to one embodiment, the anode layer may comprise a first anode sub-layer comprising or consisting of Ag or Au and a second anode-sub-layer comprising or consisting of transparent conductive oxide.

According to one embodiment, the anode layer comprises a first anode sub-layer, a second anode sub-layer and a third anode sub-layer, wherein the first anode sub-layer is arranged closer to the substrate and the second anode sub-layer is arranged closer to the cathode layer, and the third anode sub-layer is arranged between the substrate and the first anode sub-layer.

According to one embodiment, the anode layer may comprise a first anode sub-layer comprising or consisting of Ag or Au, a second anode-sub-layer comprising or consisting of transparent conductive oxide and optionally a third anode sub-layer comprising or consisting of transparent conductive oxide. Preferably the first anode sub-layer may comprise or consists of Ag, the second anode-sublayer may comprise or consists of ITO or IZO and the third anode sub-layer may comprises or consists of ITO or IZO.

Preferably the first anode sub-layer may comprise or consists of Ag, the second anode-sublayer may comprise or consists of ITO and the third anode sub-layer may comprise or consist of ITO.

Preferably, the transparent conductive oxide in the second and third anode sub-layer may be selected the same.

According to one embodiment, the anode layer may comprise a first anode sub-layer comprising Ag or Au having a thickness of 100 to 150 nm, a second anode-sub-layer comprising or consisting of a transparent conductive oxide having a thickness of 3 to 20 nm and a third anode sub-layer comprising or consisting of a transparent conductive oxide having a thickness of 3 to 20 nm.

### Hole injection layer

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the hole transport compound that is used to form the HIL, and the desired structure and thermal properties of the HIL. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the hole transport compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of a compound of formula (I).

The thickness of the HIL may be in the range from about 1 nm to about 15 nm, and for example, from about 2 nm to about 15 nm, alternatively about 2 nm to about 12 nm.

When the thickness of the HIL is within this range, the HIL may have excellent hole injecting characteristics, without a substantial penalty in driving voltage.

According to one embodiment of the present invention, the hole injection layer may comprise:
- at least about ≥ 0.5 wt.-% to about ≤ 30 wt. %, preferably about ≥ 0.5 wt.-% to about ≤ 20 wt.-%, and more preferred about ≥ 15 wt.-% to about ≤ 1 wt.-% of a compound of formula (I), and
- at least about ≥ 70 wt.-% to about ≤ 99.5 wt. %, preferably about ≥ 80 wt.-% to about ≤ 99.5 wt. %, and more preferred about ≥ 85 wt.-% to about ≤ 99 wt.-% of a substantially covalent matrix compound; preferably the wt.-% of the compound of formula (I) is lower than the wt.-% of the substantially covalent matrix compound ; wherein the weight-% of the components are based on the total weight of the hole injection layer.

Preferably, the hole injection layer may be free of ionic liquids, metal phthalocyanine, CuPc, HAT-CN, Pyrazino[2,3-f][1,10]phenanthroline-2,3-dicarbonitrile, F₄TCNQ, metal fluoride and/or metal oxides, wherein the metal in the metal oxide is selected from Re and/or Mo. Thereby, the hole injection layer may be deposited under conditions suitable for mass production.

According to an embodiment of the electronic device, wherein the hole injection layer is non-emissive.

It is to be understood that the hole injection layer is not part of the anode layer.

### Further layers

In accordance with the invention, the electronic device may comprise, besides the layers already mentioned above, further layers. Exemplary embodiments of respective layers are described in the following:

### Substrate

The substrate may be any substrate that is commonly used in manufacturing of electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a transistor backplane. Preferably, the substrate is a silicon substrate or transistor backplane.

### Hole transport layer

According to one embodiment of the electronic device, wherein the electronic device further comprises a hole transport layer, wherein the hole transport layer is arranged between the hole injection layer and the at least one first emission layer.

The hole transport layer may comprise a substantially covalent matrix compound. According to one embodiment the substantially covalent matrix compound of the hole transport layer may be selected from at least one organic compound. The substantially covalent matrix may consist substantially from covalently bound C, H, O, N, S, which optionally comprise in addition covalently bound B, P, As and/or Se.

According to one embodiment of the electronic device, the hole transport layer comprises a substantially covalent matrix compound, wherein the substantially covalent matrix compound of the hole transport layer may be selected from organic compounds consisting substantially from covalently bound C, H, O, N, S, which optionally comprise in addition covalently bound B, P, As and/or Se.

According to one embodiment, the substantially covalent matrix compound of the hole transport layer may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound of the hole injection layer and the substantially covalent matrix compound of the hole transport layer are selected the same.

According to one embodiment of the electronic device, wherein the hole transport layer of the electronic device comprises a substantially covalent matrix compound, preferably the substantially covalent matrix compound in the hole injection layer and hole transport layer are selected the same.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the hole transport compound that is used to form the HTL.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 200 nm, further about 100 nm to about 180 nm, further about 110 nm to about 140 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime may be improved. Typically, the electron blocking layer comprises a triarylamine compound.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer may be achieved. The triplet control layer may be selected from triarylamine compounds with a triplet level above the triplet level of the phosphorescent emitter in the adjacent emission layer.

The thickness of the electron blocking layer may be selected between 2 and 20 nm.

### Photoactive layer (PAL)

The photoactive layer converts an electrical current into photons or photons into an electrical current. The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL. It may be provided that the photoactive layer does not comprise the compound of Formula (I). The photoactive layer may be a light-emitting layer or a light-absorbing layer.

### Emission layer (EML)

The at least one first emission layer (EML), also referred to as first emission layer may be formed on the HTL or EBL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to the present invention it is preferred that the electronic device comprises one emission layer that is named "first emission layer". However, the electronic device optionally comprises two emission layers, wherein the first layer is named first emission layer and second layer is named second emission layer.

It may be provided that the at least one emission layer also referred to as first emission layer is free of the matrix compound of the hole injection layer.

It may be provided that the at least one emission layer does not comprise the compound of Formula (I).

The at least one emission layer (EML) may be formed of a combination of a host and an emitter dopant. Example of the host are Alq3, 4,4'-N,N'-dicarbazole-biphenyl (HTC-10), poly(n-vinyl carbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters; however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)₂Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the at least one emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent emitter dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form an HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitate injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode layer

The cathode layer is formed on the ETL or optional EIL. The cathode layer may be formed of a metal, an alloy, an electrically conductive compound, or a mixture thereof. The cathode layer may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode layer may be formed of a transparent conductive oxide, such as ITO or

### IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

### Method of manufacturing

According to another aspect of the present invention, there is provided a method of manufacturing an electronic device, the method using:
- at least one deposition source, preferably two deposition sources and more preferred at least three deposition sources.

The methods for deposition that may be suitable comprise:
- deposition via vacuum thermal evaporation;
- deposition via solution processing, preferably the processing may be selected from spin-coating, printing, casting; and/or
- slot-die coating.

According to various embodiments of the present invention, there is provided a method using:
- a first deposition source to release the matrix compound, and
- a second deposition source to release the compound of formula (I), also named metal complex.

The method comprising the steps of forming the hole injection layer; whereby for an electronic device:
- the hole injection layer is formed by releasing the matrix compound according to the invention from the first deposition source and the compound of formula (I), also named metal complex, from the second deposition source.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiment according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 1 is a schematic sectional view of an organic electronic device, according to an exemplary embodiment of the present invention;
FIG. 2 is a schematic sectional view of an organic light-emitting diode (OLED), according to an exemplary embodiment of the present invention;
FIG. 3 is a schematic sectional view of an organic light-emitting diode (OLED), according to an exemplary embodiment of the present invention.
FIG. 4 is a schematic sectional view of an organic light-emitting diode (OLED), according to an exemplary embodiment of the present invention;
FIG. 5 is a schematic sectional view of an organic light-emitting diode (OLED), according to an exemplary embodiment of the present invention.
FIG. 6 is a schematic sectional view of an organic light-emitting diode (OLED), according to an exemplary embodiment of the present invention.

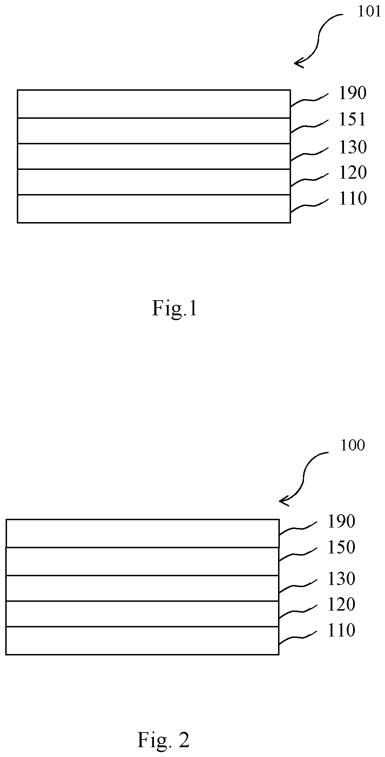

FIG. 1 is a schematic sectional view of an organic electronic device 101, according to an exemplary embodiment of the present invention. The organic electronic device 101 includes a substrate (110), an anode layer (120), a semiconductor layer comprising a compound of formula (I) (130), a photoactive layer (PAL) (151) and a cathode layer (190).

FIG. 2 is a schematic sectional view of an organic light-emitting diode (OLED) 100, according to an exemplary embodiment of the present invention. The OLED 100 includes a substrate (110), an anode layer (120), a semiconductor layer comprising a compound of formula (I) (130), an emission layer (EML) (150) and a cathode layer (190).

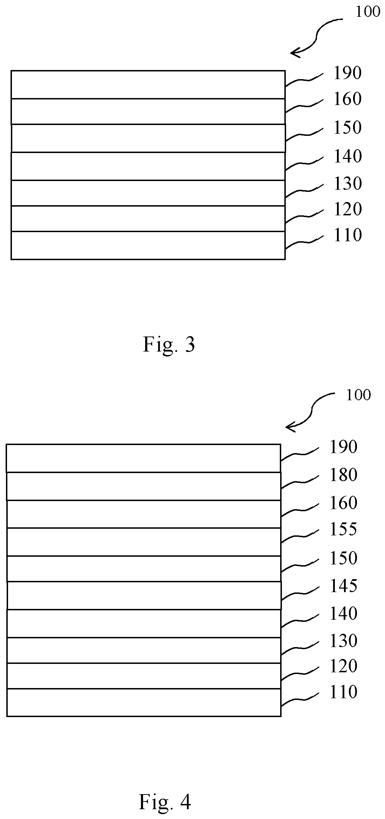

FIG. 3 is a schematic sectional view of an organic light-emitting diode (OLED) 100, according to an exemplary embodiment of the present invention. The OLED 100 includes a substrate (110), an anode layer (120), a semiconductor layer comprising a compound of formula (I) (130), a hole transport layer (HTL) (140), an emission layer (EML) (150), an electron transport layer (ETL) (160) and a cathode layer (190).

FIG. 4 is a schematic sectional view of an organic light-emitting diode (OLED) 100, according to an exemplary embodiment of the present invention. The OLED 100 includes a substrate (110), an anode layer (120), a semiconductor layer comprising a compound of formula (I) (130), a hole transport layer (HTL) (140), an electron blocking layer (EBL) (145), an emission layer (EML) (150), a hole blocking layer (HBL) (155), an electron transport layer (ETL) (160), an optional electron injection layer (EIL) (180), and a cathode layer (190).

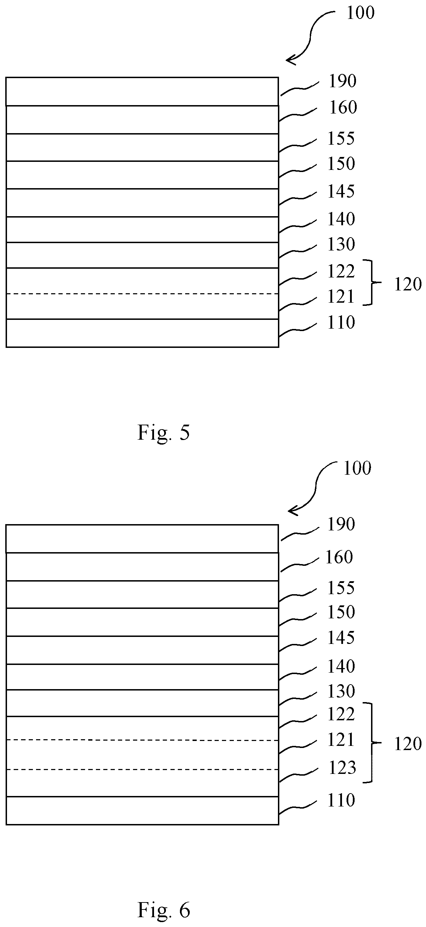

FIG. 5 is a schematic sectional view of an organic light-emitting diode (OLED) 100, according to an exemplary embodiment of the present invention. The OLED 100 includes a substrate (110), an anode layer (120) that comprises a first anode sub-layer (121) and a second anode sub-layer (122), a semiconductor layer comprising compound of formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190).

FIG. 6 is a schematic sectional view of an organic light-emitting diode (OLED) 100, according to an exemplary embodiment of the present invention. The OLED 100 includes a substrate (110), an anode layer (120) that comprises a first anode sub-layer (121), a second anode sub-layer (122) and a third anode sub-layer (123), a semiconductor layer comprising compound of formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190). The layers are disposed exactly in the order as mentioned before.

In the description above the method of manufacture an organic electronic device 101 of the present invention is for example started with a substrate (110) onto which an anode layer (120) is formed, on the anode layer (120), a semiconductor layer comprising compound of formula (I) (130), a photoactive layer (151) and a cathode electrode 190 are formed, exactly in that order or exactly the other way around.

In the description above the method of manufacture an OLED of the present invention is started with a substrate (110) onto which an anode layer (120) is formed, on the anode layer (120), a semiconductor layer comprising compound of formula (I) (130), optional a hole transport layer (140), optional an electron blocking layer (145), an emission layer (150), optional a hole blocking layer (155), optional an electron transport layer (160), optional an electron injection layer (180), and a cathode electrode 190 are formed, exactly in that order or exactly the other way around.

The semiconductor layer comprising a compound of formula (I) (130) can be a hole injection layer.

While not shown in Fig. 1, Fig. 2, Fig. 3, Fig. 4, Fig. 5 and Fig. 6, a capping layer and/or a sealing layer may further be formed on the cathode electrodes 190, in order to seal the OLEDs 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Synthesis methods

Compounds of formula (I) may be prepared as described below.

### Synthesis of 3-(2,3,5-trifluoro-6-(trifluoromethyl)pyridin-4-yl)pentane-2,4-dione

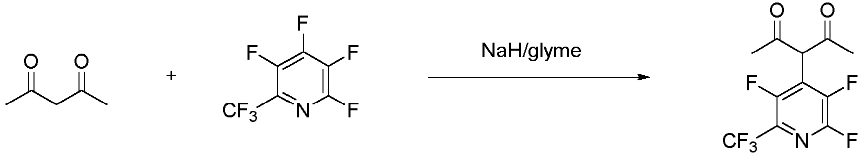

To 2.41g (100.43 mmol) sodium hydride in a flame-dried Schleck flask 200mL dry glyme was added via a double-needle cannula. The suspension was cooled with an ice-bath and 10.3mL (100.43mmol) of acetylacetone was added dropwise. During the addition the temperature should not rise above 10 °C. 20 g (91.30mmol) of 2,3,4,5-tetrafluoro-6-(trifluormethyl)pyridine was added with a syringe. The mixture was stirred at room temperature for 5 days and then added to 0.5L water, and acidified with conc. HCl to pH 1. The product was extracted with ethyl acetate. The combined organic layers were washed with water, dried over sodium sulfate, filtered and the solvent was removed under reduced pressure. The crude product was dissolved in hot methanol/water (3: 1) and after cooling the precipitate was filtered off and dried in high vacuum. Yield: 10.5g (38%)

### Synthesis of tris(((Z)-4-oxo-3-(2,3,5-trifluoro-6-(trifluoromethyl)pyridin-4-yl)pent-2-en-2-yl)oxy)iron

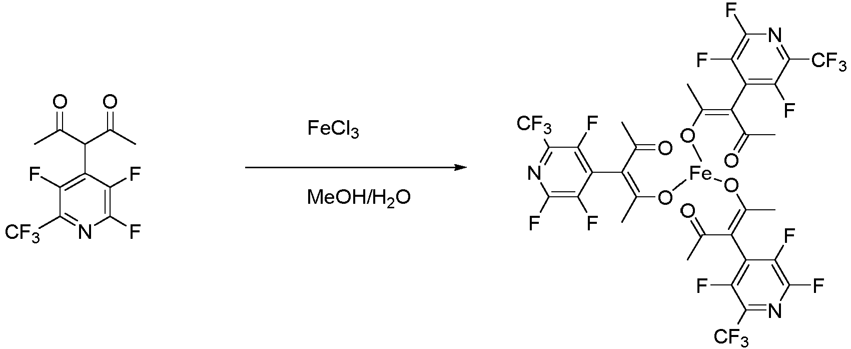

7.0g (23.4mmol) of substituted acetylacetone was dissolved in 70mL of methanol. 1.90g (23.4mmol) of sodium bicarbonate was dissolved in 20mL of water and added to the solution. The resulting suspension was heated to reflux and to the cloudy solution a solution of 1.27g (7.8 mmol) iron(III)chloride in 5mL water was added drop wise. The mixture was stirred for 30min under reflux. After cooling the residue was filtered off and washed with water. The crude product was dissolved in THF and precipitated from methanol/water, filtered off and dried in high vacuum. Yield: 5.17g (70%)

### Synthesis of bis(((Z)-4-oxo-3-(2,3,5-trifluoro-6-(trifluoromethyl)pyridin-4-yl)pent-2-en-2-yl)oxy)copper

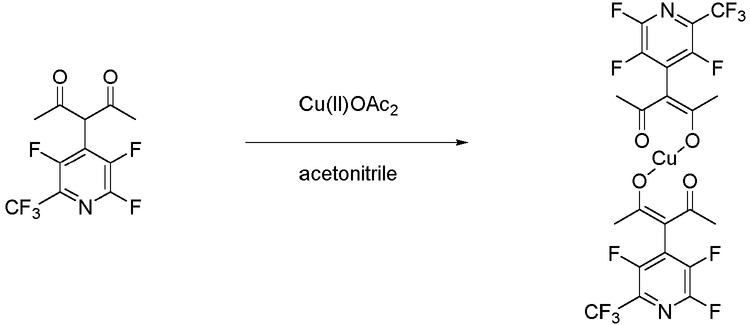

2.99g (10mmol) of substituted acetylacetone was dissolved in 50mL of acetonitrile. 1.0g (5mmol) of copper(II)acetate-monohydrate was added as a solid. To the deep blue solution 75mL of water was added and the resulting violet suspension was stirred at room temperature for 2h. The solid was filtered off and dried in vacuum. Yield: 2.95g (95%)

### Sublimation temperature

Under nitrogen in a glovebox, 0.5 to 5 g compound are loaded into the evaporation source of a sublimation apparatus. The sublimation apparatus consist of an inner glass tube consisting of bulbs with a diameter of 3 cm which are placed inside a glass tube with a diameter of 3.5 cm. The sublimation apparatus is placed inside a tube oven (Creaphys DSU 05/2.1). The sublimation apparatus is evacuated via a membrane pump (Pfeiffer Vacuum MVP 055- 3C) and a turbo pump (Pfeiffer Vacuum THM071 YP). The pressure is measured between the sublimation apparatus and the turbo pump using a pressure gauge (Pfeiffer Vacuum PKR 251). When the pressure has been reduced to 10⁻⁵ mbar, the temperature is increased in increments of 10 to 30 K till the compound starts to be deposited in the harvesting zone of the sublimation apparatus. The temperature is further increased in increments of 10 to 30 K till a sublimation rate is achieved where the compound in the source is visibly depleted over 30 min to 1 hour and a substantial amount of compound has accumulated in the harvesting zone. The sublimation temperature, also named T_{subl}, is the temperature inside the sublimation apparatus at which the compound is deposited in the harvesting zone at a visible rate and is measured in degree Celsius.

### Rate onset temperature

The rate onset temperature (T_{RO}) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10⁻⁵ mbar and the temperature inside the source measured with a thermocouple. Evaporation of the compound is detected with a QCM detector which detects deposition of the compound on the quartz crystal of the detector. The deposition rate on the quartz crystal is measured in Ǻngstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

### General procedure for fabrication of electronic devices comprising a semiconductor layer

### comprising a metal complex and a matrix compound

For inventive examples 1 to 3 and comparative examples 1 to 3 in Table 2, a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment to prepare the anode layer. The plasma treatment was performed in an atmosphere comprising 97.6 vol.-% nitrogen and 2.4 vol.-% oxygen at 75 W for 35 seconds.

Then, the matrix compound and the metal complex were co-deposited in vacuum on the anode layer, to form a hole injection layer (HIL) having a thickness of 10 nm. The composition of the hole injection layer can be seen in Table 2. In inventive examples 1 to 3, a compound of formula (I) is used.

### Matrix compound HTM-1 has the following formula:

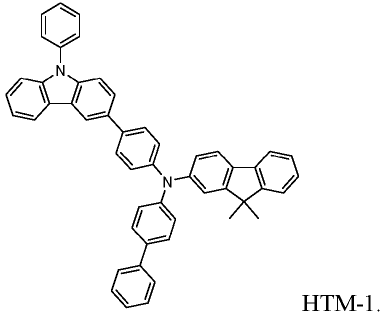

Then, the matrix compound was vacuum deposited on the HIL, to form an HTL having a thickness of 123 nm. The matrix compound in the HTL is selected the same as the matrix compound in the HIL.

Then N-([1,1'-biphenyl]-4-yl)-9,9-diphenyl-N-(4-(triphenylsilyl)phenyl)-9H-fluoren-2-amine was vacuum deposited on the HTL, to form an electron blocking layer (EBL) having a thickness of 5 nm.

Then 97 vol. -% H09 as EML host and 3 vol. -% BD200 (Sun Fine Chemicals, Korea) as fluorescent blue emitter dopant was deposited on the EBL, to form a blue-emitting first emission layer (EML) with a thickness of 20 nm.

Then a hole blocking layer was formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer EML.

Then the electron transporting layer having a thickness of 31 nm was formed on the hole blocking layer by depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% of LiQ.

Then Ag:Mg (90:10 vol.-%) was evaporated at a rate of 0.01 to 1 Å/s at 10⁻⁷ mbar to form a cathode layer with a thickness of 13 nm on the electron transporting layer.

Then, HTM-1 was deposited on the cathode layer to form a capping layer with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm² is determined by interpolating the luminance-voltage and current-voltage characteristics, respectively.

Lifetime LT of the device is measured at ambient conditions (20°C) and 30 mA/cm², using a Keithley 2400 sourcemeter, and recorded in hours.

The brightness of the device is measured using a calibrated photo diode. The lifetime LT is defined as the time till the brightness of the device is reduced to 97 % of its initial value.

To determine the voltage stability over time U(100h)-(1h), a current density of at 30 mA/cm² was applied to the device. The operating voltage was measured after 1 hour and after 100 hours, followed by calculation of the voltage stability for the time period of 1 hour to 100 hours.

### Technical effect Table 1

In Table 1 are shown physical properties of compounds of formula (I), see inventive compounds 1 to 4, and of comparative compounds 1 to 6.

As can be seen in Table 1, the sublimation temperature of comparative compounds 1 to 6 can either not be measured due to decomposition of the compound or the sublimation temperature is in the range of 95 to 120 °C.

The rate onset temperature of comparative compounds 1 to 6 is the range of < 100 to 101 °C, see Table 1.

Inventive compound 1 is a Cu(II) complex of formula (I). Inventive compound 1 differs from comparative compound 1 in the substituted heteroaryl substituent. The sublimation temperature is increased from 110 - 120 °C in comparative compound 1 to 186 °C in inventive compound 1. The rate onset temperature is also improved to 105 °C.

Inventive compound 2 is a Fe(III) complex of formula (I). The sublimation temperature is 182 °C. The rate onset temperature is further improved to 128 °C.

Inventive compound 3 is a Fe(III) complex of formula (I). It differs from inventive compound 2 in the substituents on the heteroaryl group. The sublimation temperature is further increased to 209 °C and the rate onset temperature is further improved to 146 °C.

Inventive compound 4 is a Fe(III) complex of formula (I). It differs from inventive compound 2 and 3 in the substituted heteroaryl substituent. The sublimation temperature is still high at 182 °C and the rate onset temperature is high at 122 °C.

In summary, the thermal stability, sublimation temperature and/or rate onset temperature of compounds of formula (I) is substantially improved over the state of the art.

### OLED performance data Table 2

In Table 2 are shown OLED performance data for an increase in operating voltage over time U(100 h)-U(1h) and lifetime LT97 for inventive examples 1 to 3 and comparative examples 1 to 3.

In comparative example 1, the semiconductor layer comprises 3 vol.-% metal complex La(fod)₃. The increase in operating voltage over time is 1.07 V. The lifetime is 30 h.

In inventive example 1, the semiconductor layer comprises 3 vol.-% G6. The increase in operating voltage over time is 0.2 V. The lifetime is 75 h.

In comparative example 2, the semiconductor layer comprises 5 vol.-% metal complex La(fod)₃. The increase in operating voltage over time is 0.85 V. The lifetime is 24 h.

In inventive example 2, the semiconductor layer comprises 5 vol.-% G6. The increase in operating voltage over time is 0.3 V. The lifetime is 95 h.

In comparative example 3, the semiconductor layer comprises 10 vol.-% metal complex La(fod)₃. The increase in operating voltage over time is 0.89 V. The lifetime is 15 h.

In inventive example 3, the semiconductor layer comprises 10 vol.-% G6. The increase in operating voltage over time is 0.09 V. The lifetime is 79 h.

In summary, in a semiconductor layer comprising a compound of formula (I) the increase in operating voltage is substantially reduced and the lifetime substantially increased compared to the state of the art.

A reduced increase in operating voltage over time is an indication for improved stability of the electronic device. An increase in lifetime is important for improved stability of the electronic device.

**Table 1: Properties of comparative compounds 1 to 6 and compounds of formula (I)**

| Example | Compound | Sublimation temperature Tsubl, [°C] | Rate onset temperature T_{RO}, [°C] |
|---|---|---|---|
| Comparative compound 1 | Cu(acac)2 | 110-120 | < 100 |
| Comparative compound 2 | Cu(tfac)2 | 95-100 | < 100 |
| Comparative compound 3 | Bi(tfac)3 | decomposition | < 100 |
| Comparative compound 4 | Bi(hfac)3 | decomposition | < 100 |
| Comparative compound 5 | Bi(fod)3 | 120 | < 100 |
| Comparative compound 6 | La(fod)₃ | | 101 |
| Inventive compound 1 | G21 | 186 | 105 |
| Inventive compound 2 | G6 | 182 | 128 |
| Inventive compound 3 | G5 | 209 | 146 |
| Inventive compound 4 | G13 | 182 | 122 |

**Table 2: Performance of an electroluminescent device comprising a metal complex**

| | Metal complex | Percentage metal complex in semiconductor layer [vol.-%] | Matrix compound | Percentage matrix compound in semiconductor layer [vol.-%] | U(100 h)-U(1h) (30mA/ cm²) [V] | LT97 RT (30mA/ cm²) [h] |
|---|---|---|---|---|---|---|
| Comparative example 1 | La(fod)₃ | 3 | HTM-1 | 97 | 1.07 | 30 |
| Inventive example 1 | G6 | 3 | HTM-1 | 97 | 0.20 | 75 |
| Comparative example 2 | La(fod)₃ | 5 | HTM-1 | 95 | 0.85 | 24 |
| Inventive example 2 | G6 | 5 | HTM-1 | 95 | 0.30 | 95 |
| Comparative example 3 | La(fod)₃ | 10 | HTM-1 | 90 | 0.89 | 15 |
| Inventive example 3 | G6 | 10 | HTM-1 | 90 | 0.09 | 79 |

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. A compound represented by Formula I: 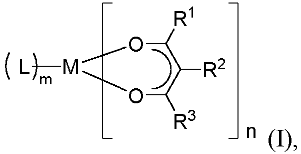 wherein
M is a metal;
L is a charge-neutral ligand, which coordinates to the metal M;
n is an integer selected from 1 to 4, which corresponds to the oxidation number of M; m is an integer selected from 0 to 2;
R¹, R² and R³ are independently selected from H, D, substituted or unsubstituted C₁ to C₁₂ alkyl, substituted or unsubstituted C₁ to C₁₂ alkoxy, substituted or unsubstituted C₆ to C₂₄ aryl, and substituted or unsubstituted C₂ to C₂₄ heteroaryl group, wherein
the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy, substituted or unsubstituted C₆ to C₁₈ aryl, and substituted or unsubstituted C₂ to C₁₈ heteroaryl, wherein
the substituents are selected from halogen, F, Cl, CN, C₁ to C₆ alkyl, CF₃, OCH₃ and OCF₃;
wherein
at least one R¹, R² and/or R³ is selected from a substituted C₂ to C₂₄ heteroaryl group, wherein at least one substituent is selected from halogen, F, Cl, CN, partially or fully fluorinated C₁ to C₆ alkyl, partially or fully fluorinated C₁ to C₆ alkoxy.

2. The compound according to claim 1, wherein the metal M is selected from alkali, alkaline earth, transition, rare earth metal or group III to V metal, preferably the metal M is selected from transition or group III to V metal; preferably the metal M is selected from Li(I), Na(I), K(I), Cs(I), Mg(II), Ca(II), Sr(II), Ba(II), Sc(III), Y(III), Ti(IV), V(III-V), Cr(III-VI), Mn(II), Mn(III), Fe(II), Fe(III), Co(II), Co(III), Ni(II), Cu(I), Cu(II), Zn(II), Ag(I), Au(I), Au(III), Al(III), Ga(III), In(III), Sn(II), Sn(IV), or Pb(II); preferably M is selected from Cu(II), Fe(III), Co(III), Mn(III), Ir(III), Bi(III); and more preferred M is selected from Fe(III) and Cu(II).

3. The compound according to claim 1 or 2, wherein L is selected from the group comprising H₂O, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (II); 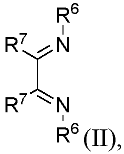 wherein R⁶ and R⁷ are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

4. The compound according to any of claims 1 to 3, wherein n is an integer selected from 1, 2 and 3, which corresponds to the oxidation number of M.

5. The compound according to any of claims 1 to 4, wherein m is an integer selected from 0 or 1, preferably 0.

6. The compound according to any of claims 1 to 5, wherein at least one R¹, R² or R³ is selected from a substituted C₂ to C₂₄ heteroaryl group, wherein
- the substituted heteroaryl group comprises at least one six-membered ring; and/or
- the substituted heteroaryl group comprises at least 1 to 6 N atoms, preferably 1 to 3 N atoms, and more preferred 1 N atom; and/or
- the heteroaryl group of the substituted heteroaryl group is a six-membered ring, comprise 1, 2 or 3 hetero atoms, wherein preferably the hetero atom is N.

7. The compound according to any of claims 1 to 6, wherein at least one R¹, R² or R³ is selected from a substituted C₂ to C₂₄ heteroaryl group, wherein the C₂ to C₂₄ heteroaryl group is selected from pyridyl, pyrimidinyl, pyrazinyl, triazinyl.

8. The compound according to any of claims 1 to 7, wherein at least one R¹, R² or R³ is selected from a substituted C₂ to C₂₄ heteroaryl group, wherein the at least one substituent of the substituted C₂ to C₂₄ heteroaryl group is selected from the group comprising halogen, F, Cl, CN, partially or fully fluorinated C₁ to C₆ alkyl, partially or fully fluorinated C₁ to C₆ alkoxy; preferably from the group comprising halogen, F, Cl, CN, partially or fully fluorinated C₁ to C₆ alkyl, partially or fully fluorinated C₁ to C₆ alkoxy; further preferred from the group comprising halogen, F, Cl, CN, partially or fully fluorinated C₁ to C₄ alkyl, partially or fully fluorinated C₁ to C4 alkoxy; and more preferred from the group comprising F, CN, partially or fully fluorinated C₁ to C₆ alkyl; also preferred F, CN, partially or fully fluorinated C₁ to C₆ alkyl, also preferred halogen, F, Cl, CN, partially or fully fluorinated C₁ to C₄ alkyl; and more preferred from the group comprising at least one CN, at least one CF₃ group, and/or at least two F atoms.

9. The compound according to any of claims 1 to 8, wherein one R¹, R² or R³ is selected from a substituted C₂ to C₂₄ heteroaryl group, wherein at least one substituent is selected from halogen, F, Cl, CN, partially or fully fluorinated C₁ to C₆ alkyl, partially or fully fluorinated C₁ to C₆ alkoxy., and one R¹, R² or R³ is selected from substituted or unsubstituted C₁ to C₁₂ alkyl or substituted or unsubstituted C₁ to C₁₂ alkoxy, wherein the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy; and one R¹, R² or R³ is selected from H, D, substituted or unsubstituted C₁ to C₁₂ alkyl, substituted or unsubstituted C₁ to C₁₂ alkoxy, wherein the at least one substituent is selected from halogen, F, Cl, CN, substituted or unsubstituted C₁ to C₆ alkyl, partially or fully fluorinated C₁ to C₆ alkyl, substituted or unsubstituted C₁ to C₆ alkoxy, partially or fully fluorinated C₁ to C₆ alkoxy; wherein
the substituents are selected from halogen, F, Cl, CN, C₁ to C₆ alkyl, CF₃, OCH₃ and OCF₃.

10. The compound according to any of claims 1 to 9, wherein at least one substituted C₂ to C₂₄ heteroaryl group of R¹, R² or R³ is selected from the following Formulas D1 to D29: 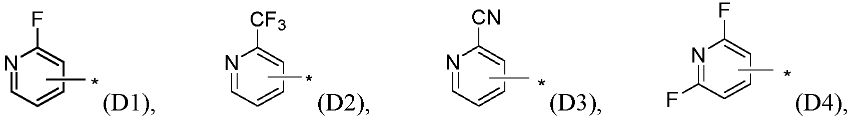 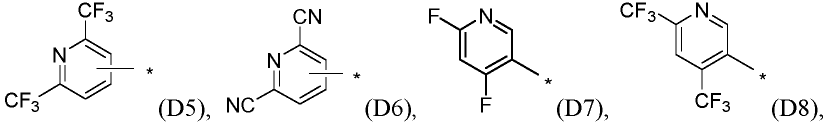 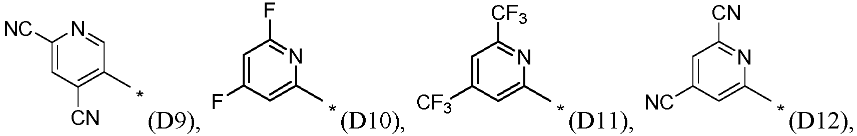 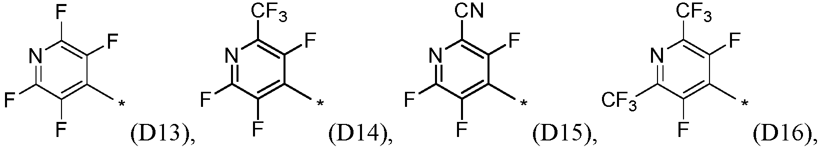 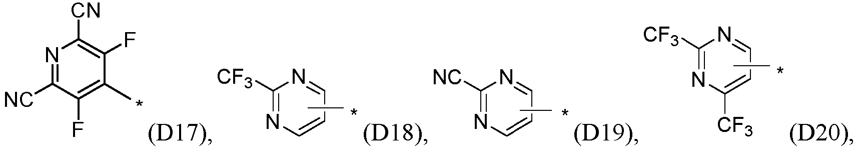 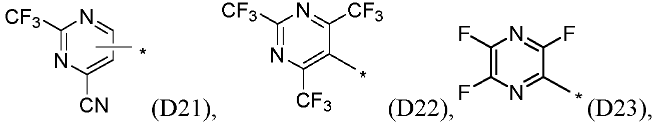 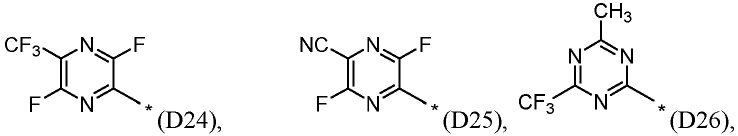 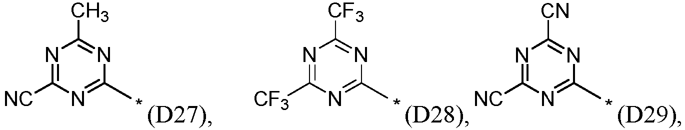 wherein the "*" denotes the binding position.

11. The compound according to any of claims 1 to 10, wherein the compound represented by Formula I is selected from the following Formulas E1 to E28:

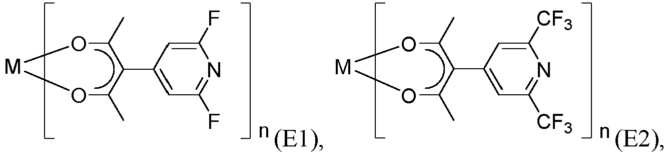

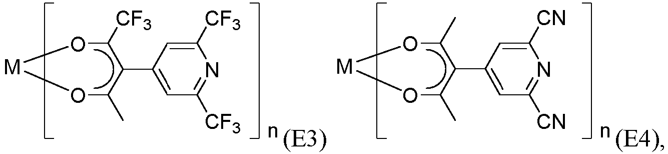

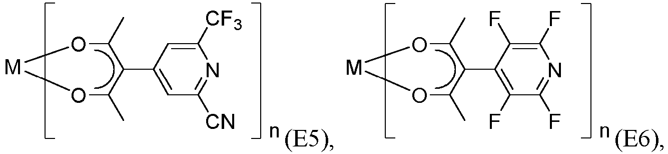

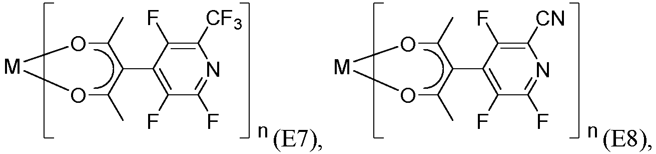

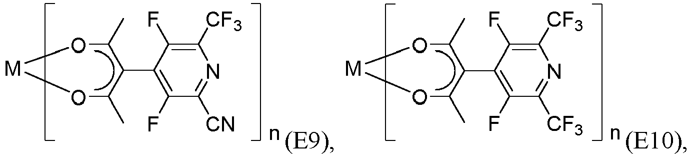

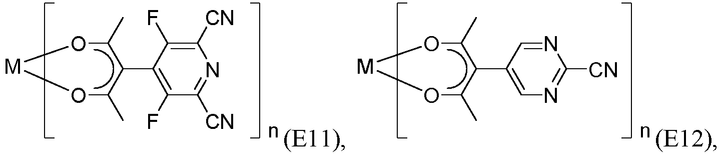

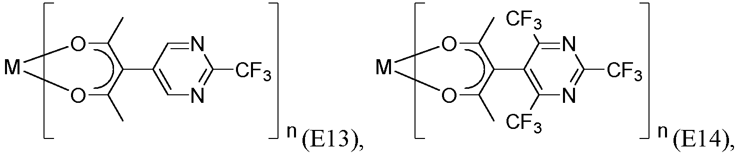

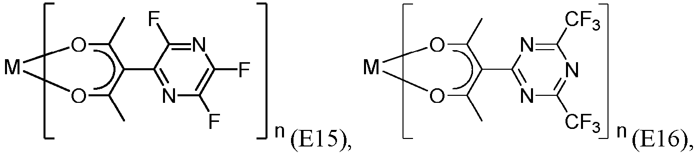

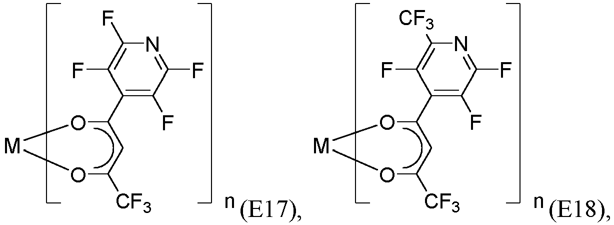

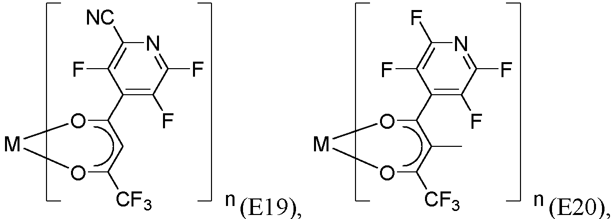

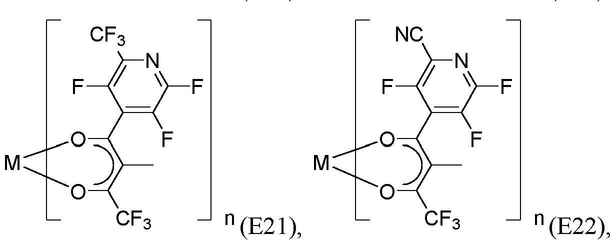

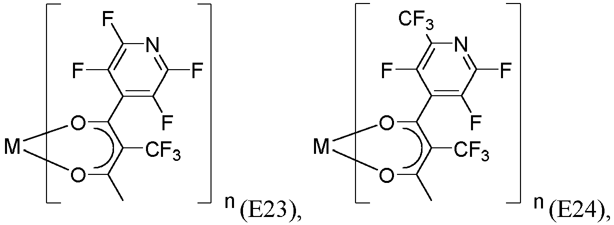

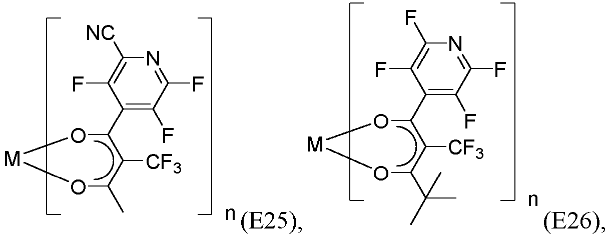

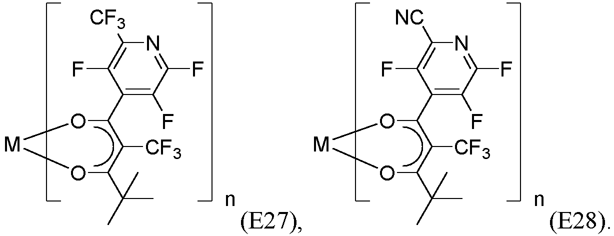

12. A semiconductor material comprising at least one compound of Formula I according to any of the preceding claims 1 to 11.

13. A semiconductor material according to claim 12, wherein the material comprises in addition at least one substantially covalent matrix compound.

14. An semiconductor layer comprising a compound of Formula I according to any of the preceding claims 1 to 13.

15. An electronic device comprising a semiconductor material according to any of the preceding claim 12 to 14.

16. The electronic device according to claim 15, wherein the electronic device is a light emitting device, thin film transistor, a battery, a display device or a photovoltaic cell, and preferably a light emitting device, preferably the electronic device is part of a display device or lighting device.
